(19) Europäisches Patentamt
European Patent Office
Office européen des brevets

(11) Veröffentlichungsnummer: **0 541 041 A1**

## (12) EUROPÄISCHE PATENTANMELDUNG

(21) Anmeldenummer: **92118813.2**

(22) Anmeldetag: **03.11.92**

(51) Int. Cl.$^5$: **C07D 239/60**, C07D 251/30, C07D 401/12, C07D 405/12, C07F 7/18, C07F 7/08, C07F 9/6506, C07F 9/6521, A01N 43/54

(30) Priorität: **07.11.91 DE 4136569**

(43) Veröffentlichungstag der Anmeldung: **12.05.93 Patentblatt 93/19**

(84) Benannte Vertragsstaaten: **AT BE CH DE DK ES FR GB IT LI NL SE**

(71) Anmelder: **HOECHST AKTIENGESELLSCHAFT**
**Postfach 80 03 20**
**W-6230 Frankfurt am Main 80(DE)**

(72) Erfinder: **Zurmühlen, Frank, Dr.**
**Inselsbergstrasse 13**
**W-6230 Frankfurt am Main 80(DE)**
Erfinder: **Bauer, Klaus, Dr.**
**Doorner Strasse 53d**
**W-6450 Hanau(DE)**
Erfinder: **Bieringer, Hermann, Dr.**
**Eichenweg 26**
**W-6239 Eppstein/Ts.(DE)**

(54) **Pyrimidinyl- oder Triazinyl-oxy-(oder -thio)-carbonsäurederivate, Verfahren zu ihrer Herstellung und Verwendung als Herbizide oder Pflanzenwachstumsregulatoren.**

(57) Verbindungen der Formel (I)

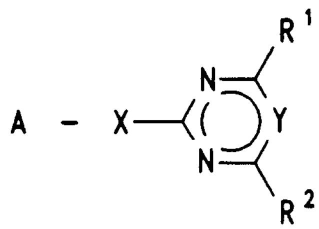

(I)

worin
A

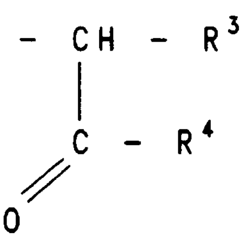

oder

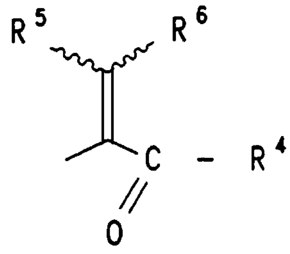

ist



Rank Xerox (UK) Business Services
(3.10/3.6/3.3.1)

und $R^1$, $R^2$, $R^3$, $R^4$, $R^5$, $R^6$, X und Y wie in Anspruch 1 definiert sind, eignen sich als selektive Herbizide und Pflanzenwachstumsregulatoren.

Es ist bekannt, daß Pyrimidinyloxy(thio)- und Triazinyloxy(thio)-carbonsäurederivate herbizide und pflanzenwachstumsregulierende Eigenschaften aufweisen (s. EP-A-0347811, EP-A-0409369 (CA-A-2021486), EP-A-0409368 (CA-A-2021486), EP-A-400741). Die bekannten Wirkstoffe dieses Strukturtyps weisen jedoch zum Teil bei ihrer Anwendung Nachteile auf, wie beispielsweise unzureichende Selektivität in wichtigen Nutzkulturen.

Es wurden nun neue Pyrimidinyloxy(thio)- und Triazinyloxy(thio)carbonsäurederivate mit vorteilhaften herbiziden Eigenschaften gefunden.

Gegenstand der Erfindung sind Verbindungen der allgemeinen Formel (I),

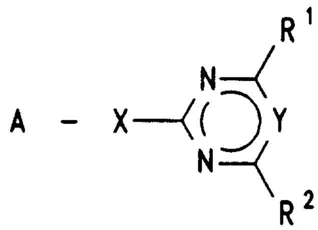

(I)

worin

A einen Rest der Formel

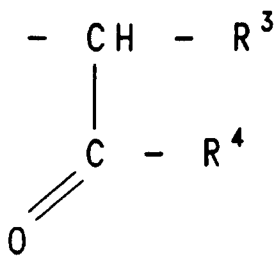

oder

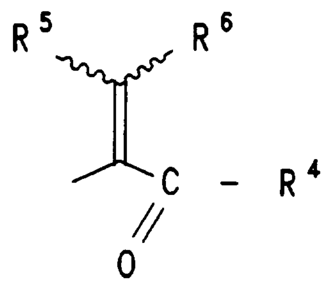

X O oder S,

Y N oder CH,

$R^1$,$R^2$ unabhängig voneinander Wasserstoff, Alkyl, Alkoxy, Alkylthio, Halogen, Haloalkoxy, Amino, Alkylamino oder Dialkylamino,

$R^3$ einen aliphatischen, araliphatischen oder aromatischen Rest der Formel

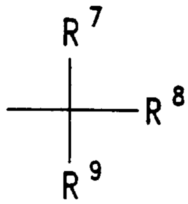 , 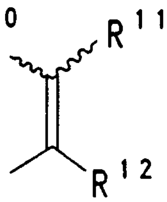 , 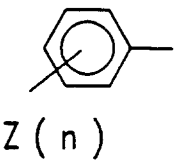 , 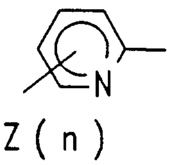

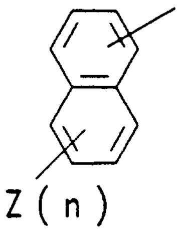 , 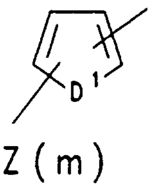 , 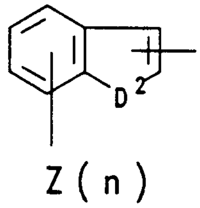 oder 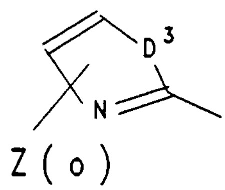

$R^5$ Alkyl, Alkenyl oder Alkinyl, wobei die letztgenannten drei Reste unabhängig voneinander unsubstituiert oder ein- oder mehrfach durch Reste aus der Gruppe Alkoxy, Alkylthio, Amino, Alkylamino, Dialkylamino, Nitro, Halogen, Phenyl und substituiertes Phenyl substituiert sind, oder Cycloalkyl oder Cycloalkenyl, wobei die letztgenannten beiden Reste unabhängig voneinander unsubstituiert oder ein- oder mehrfach durch Reste aus der Gruppe Alkyl, Alkoxy, Alkylthio, Amino, Alkylamino, Dialkylamino, Nitro, Halogen, Phenyl und substituiertes Phenyl substituiert sind, oder einen Rest der Formel

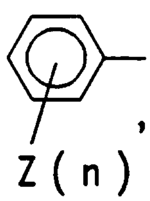 , 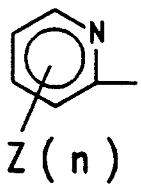 , 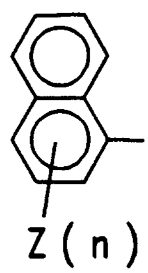 , 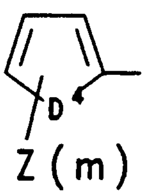 , 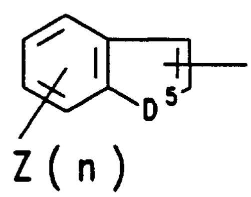

oder 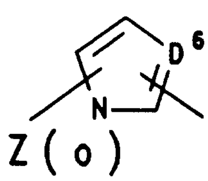 ,

$R^6$ Wasserstoff oder Alkyl,

$R^7$ Wasserstoff, Halogen, Alkyl, das unsubstituiert oder durch einen oder mehrere Reste aus der Gruppe Halogen, Hydroxy, Alkoxy und Alkylthio substituiert ist, oder Cycloalkyl, Hydroxy, Cyano, Thienyl, Naphthyl, Dihydronaphthyl, wobei die letztgenannten drei Reste unsubstituiert oder substituiert sind, oder einen Rest der Formel

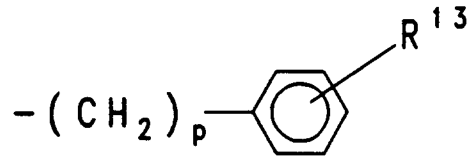

| | |
|---|---|
| $R^{13}$ | Wasserstoff, Halogen, Nitro, Alkyl, Haloalkyl, Alkoxy, Alkylsulfonyl, Alkylsulfinyl oder Alkylthio, |
| $R^8$,$R^9$ | unabhängig voneinander Wasserstoff oder Alkyl oder $R^8$ und $R^9$ gemeinsam mit dem verbundenen C-Atom einen 3- bis 6-gliedrigen Ring, der ein Sauerstoffatom enthalten kann und durch ein oder mehrere Alkylgruppen substituiert sein kann, |
| $R^{10}$ | unabhängig voneinander Wasserstoff oder Alkyl oder $R^{10}$ gemeinsam mit $R^{12}$ und der verbundenen Ethenylen-Gruppe einen Cyclopenten-oder Cyclohexenring, der unsubstituiert oder durch ein oder mehrere Alkylgruppen substituiert ist, |
| $R^{11}$ | Wasserstoff oder Alkyl, |
| $R^{12}$ | Alkyl, Phenyl oder substituiertes Phenyl oder $R^{12}$ gemeinsam mit $R^{10}$ und der verbundenen Ethenylen-Gruppe einen Cyclopenten-oder Cyclohexenring, der unsubstituiert oder durch ein oder mehrere Alkylgruppen substituiert ist, |
| $D^1$ bis $D^6$ | unabhängig voneinander O, S oder $NR^{14}$, |
| $R^{14}$ | Wasserstoff, Alkyl oder Phenyl, |
| n | eine ganze Zahl von 0 bis 4, vorzugsweise 0 bis 3, |
| m | eine ganze Zahl von 0 bis 3, vorzugsweise 0 oder 1, |
| o | eine ganze Zahl von 0 bis 2, vorzugsweise 0 oder 1, |
| p | eine ganze Zahl von 0 bis 2, vorzugsweise 0 oder 1, |
| Z | unabhängig voneinander Halogen, Alkyl, Haloalkyl, Alkoxy, Haloalkoxy, Alkylthio, Amino, Alkylamino, Dialkylamino, Nitro, Phenyl oder substituiertes Phenyl, |
| $R^4$ | einen Alkoxyrest, der durch Alkenyloxy, Alkinyloxy, Cycloalkyloxy, Cycloalkenyloxy, Benzyloxy, Benzylthio, $(R^*)_3Si$ oder $(R^*)_3SiO$, worin die Reste $R^*$ unabhängig voneinander für Alkyl, Alkenyl oder Alkinyl, Aryl oder substituiertes Aryl stehen, oder Cyano, Nitro, Alkylcarbonyl, Alkenylcarbonyl, Alkinylcarbonyl, Cycloalkylcarbonyl, Alkylsulfonyl, Alkylsulfinyl, Dialkylphosphinyl, Dialkylphosphonyl, Dialkylphosphoryl, Pyridyl oder substituiertes Pyridyl substituiert ist, oder<br>$R^4$ den Rest eines 5- oder 6-Ringlactons oder<br>$R^4$ einen Rest der Formel |

$-D^7-CR^{15}R^{16}-D^8-L$
$-NR^{32}-SO_2-R^{33}$
$-NR^{34}-O-R^{35}$
$-NH-N=CR^{21}R^{22}$ oder

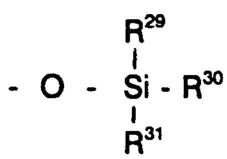

| | |
|---|---|
| $D^7$ | O, S oder $NR^{14}$, |
| $D^8$ | eine direkte Bindung oder eine gesättigte oder ungesättigte Kohlenwasserstoffgruppe, vorzugsweise eine direkte Bindung, $CH_2$, $CH(CH_3)$ oder $C(CH_3)_2$, |
| $R^{15}$, $R^{16}$ | unabhängig voneinander Wasserstoff, Alkyl, Alkenyl, Alkinyl, Aryl, substituiertes Aryl, Benzyl, Alkoxy, Alkenyloxy, Alkinyloxy, Aryloxy oder substituiertes Aryloxy, |
| L | einen Säurederivat-Rest der Formel |

$-CO-O-R^{17}$
$-CO-S-R^{18}$
$-CO-NR^{19}R^{20}$

$-CO-O-N=CR^{21}R^{22}$
$-CO-N=CR^{21}R^{22}$
$-O-CO-R^{23}$
$-S-CO-R^{23}$
$-NR^{14}-CO-R^{23}$

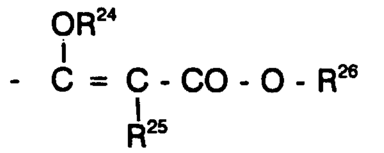 oder

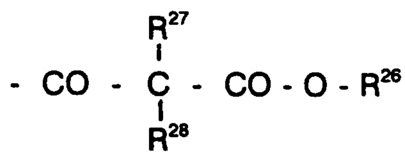

| | |
|---|---|
| $R^{17}$, $R^{18}$ | Wasserstoff, Alkyl, Alkenyl oder Alkinyl, wobei die letztgenannten 3 Reste unsubstituiert oder durch einen oder mehrere Reste aus der Gruppe Halogen substituiert sind, Cycloalkyl, Cycloalkenyl, Benzyl, Phenyl oder substituiertes Phenyl, |
| $R^{19}$, $R^{20}$ | unabhängig voneinander Wasserstoff, Alkyl, Alkenyl, Alkinyl, Cycloalkyl, Benzyl, Phenyl oder substituiertes Phenyl oder gemeinsam mit dem verbundenen N-Atom einen heterocyclischen 3-bis 7-gliedrigen Ring, der neben dem N-Atom noch Heteroatome aus der Gruppe N, O und S enthalten kann, |
| $R^{21}$, $R^{22}$ | unabhängig voneinander Wasserstoff, Alkyl, Alkenyl oder Alkinyl oder gemeinsam mit dem verbundenen C-Atom einen carbocylischen 4-bis 8-gliedrigen Rest, |
| $R^{23}$ | Wasserstoff, Alkyl, Cycloalkyl, Phenyl oder substituiertes Phenyl, |
| $R^{24}$ | Alkyl, Alkenyl oder Alkinyl, wobei die letztgenannten 3 Reste unsubstituiert oder durch einen oder mehrere Reste aus der Gruppe Halogen, Alkoxy, Alkenyloxy und Alkinyloxy substituiert sind, oder Cycloalkyl, Cycloalkenyl, Alkanoyl, Alkoxycarbonyl, Benzyl, Benzoxycarbonyl, Phenyl oder Benzoyl, wobei die letztgenannten 4 Reste unsubstituiert oder substituiert sind, |
| $R^{25}$ | Wasserstoff, Alkyl, Alkanoyl, Alkoxycarbonyl, Alkenyloxycarbonyl, Alkinyloxycarbonyl, Phenyl oder substituiertes Phenyl, |
| $R^{26}$ | Wasserstoff, Alkyl, Alkenyl oder Alkinyl, wobei die letztgenannten 3 Reste unsubstituiert oder durch einen oder mehrere Reste aus der Gruppe Halogen substituiert sind, |
| $R^{27}$, $R^{28}$ | unabhängig voneinander Wasserstoff, Alkyl, Alkanoyl, Alkoxycarbonyl, Alkenyloxycarbonyl, Alkinyloxycarbonyl, Phenyl oder substituiertes Phenyl, |
| $R^{29}$, $R^{30}$, $R^{31}$ | unabhängig voneinander Alkyl, Alkenyl oder Alkinyl, Phenyl oder substituiertes Phenyl, |
| $R^{32}$ | Wasserstoff, Alkyl, Alkenyl, Alkinyl, Aryl oder Aralkyl, |
| $R^{33}$ | Alkyl, Aryl, substituiertes Aryl, Aralkyl oder substituiertes Aralkyl, |
| $R^{34}$ | Wasserstoff, Alkyl, Alkenyl, Alkinyl, Aryl oder Aralkyl und |
| $R^{35}$ | Wasserstoff, Alkyl, Alkenyl, Alkinyl, Aryl oder Aralkyl |

bedeuten.

In der Formel (I) und im folgenden können die Reste Alkyl, Alkoxy, Haloalkyl, Haloalkoxy, Alkylamino und Alkylthio sowie die entsprechenden ungesättigten und/oder substituierten Reste jeweils geradkettig oder verzweigt sein. Wenn nicht speziell angegeben, sind bei diesen Resten die Kohlenstoffatomketten oder Kohlenstoffgerüste mit 1 bis 4 C-Atomen, bzw. bei ungesättigten Gruppen mit 2 bis 4 C-Atomen bevorzugt. Alkylreste, auch in den zusammengesetzten Bedeutungen wie Alkoxy, Haloalkyl usw., bedeuten Methyl, Ethyl, n- oder i-Propyl, n-, i-, t- oder 2-Butyl; Alkenyl- und Alkinylreste haben die Bedeutung der den Alkylresten entsprechenden möglichen ungesättigten Reste, wie 2-Propenyl, 2- oder 3-Butenyl, 2-Propinyl, 2- oder 3-Butinyl. Halogen bedeutet Fluor, Chlor, Brom oder Jod, Haloalkyl bedeutet Alkyl, das durch ein oder mehrere Atome aus der Gruppe Halogen substituiert ist; Haloalkyl ist beispielsweise $CF_3$, $CHF_2$, $CH_2CF_3$. Entsprechendes gilt für Haloalkenyl, Haloalkoxy und andere durch Halogen substituierte Reste. Aryl bedeutet beispielsweise Phenyl, Naphthyl, Tetrahydronaphthyl, Indenyl,

Indanyl, Pentalenyl, Fluorenyl und ähnliches, vorzugsweise Phenyl; Aryloxy bedeutet die entsprechenden Oxy-Reste, vorzugsweise Phenoxy. Heteroaryl bzw. Heteroaryl in Heteroaryloxy bedeutet beispielsweise Pyridyl, Pyrimidyl, Pyridazyl, Thienyl, Furyl, Pyrrolyl, Pyrazolyl und Imidazolyl, vorzugsweise Pyridyl und Thienyl, aber auch bicyclische oder polycyclische aromatische oder araliphatische Verbindungen. Gegebenenfalls substituiertes Aryl, Aryloxy, Heteroaryl, Heteroaryloxy, Phenyl, Phenoxy, Benzyl und Benzyloxy sowie bicyclische Reste mit aromatischen Anteilen bedeuten vorzugsweise jeweils den entsprechenden unsubstituierten Rest oder bedeuten einen davon abgeleiteten substituierten Rest, wobei die Substituenten beispielsweise einen oder mehrere, vorzugsweise 1, 2 oder 3 Reste aus der Gruppe Halogen, Alkyl, Haloalkyl, Alkoxy, Haloalkoxy, Nitro, Cyano, Alkoxycarbonyl, Alkylcarbonyl, Alkanoyl, Carbamoyl, Mono- und Dialkylaminocarbonyl, Mono- und Dialkylamino, Alkylsulfinyl und Alkylsulfonyl bedeuten und bei Resten mit C-Atomen solche mit 1 bis 4 C-Atomen, insbesondere 1 oder 2, bevorzugt sind. Bevorzugt sind in der Regel Substituenten aus der Gruppe Halogen, wie Fluor und Chlor, $C_1-C_4$-Alkyl, vorzugsweise Methyl oder Ethyl, $C_1-C_4$-Haloalkyl, vorzugsweise Trifluormethyl, $C_1-C_4$-Alkoxy, vorzugsweise Methoxy oder Ethoxy, $C_1-C_4$-Haloalkoxy, Nitro und Cyano. Besonders bevorzugt sind Methyl, Methoxy und Chlor.

Beispiele für siliciumhaltige Reste $R^4$ sind Trialkylsilylalkoxy, Aryldialkylsilylalkoxy, Diarylalkylsilylalkoxy, Trialkylsilylalkenyloxy, Aryldialkylsilylalkenyloxy, Diarylalkylsilylalkenyloxy, Trialkylsilylalkinyloxy, Aryldialkylsilylalkinyloxy, Diarylalkylsilylalkinyloxy, Trialkylsilyloxy, Aryldialkylsilyloxy, Diarylalkylsilyloxy, Gegenstand der Erfindung sind auch alle Stereoisomeren und deren Gemische, die von Formel (I) umfaßt, jedoch nicht spezifisch definiert sind.

Von besonderem Interesse sind erfindungsgemäße Verbindungen der Formel (I), worin

$R^1$, $R^2$ unabhängig voneinander $C_1-C_4$-Alkyl, $C_1-C_4$-Alkoxy, Halogen, $C_1-C_4$-Haloalkoxy, $C_1-C_4$-Alkylamino oder Di-($C_1-C_4$-alkyl)amino, vorzugsweise Methyl, Methoxy, Chlor oder Methylamino, insbesondere einer von beiden Resten Methoxy und der andere Rest Methyl, Methoxy, Chlor oder Methylamino,

bedeuten.

Von besonderem Interesse sind auch erfindungsgemäße Verbindungen der Formel (I), worin

A einen Rest der Formel $-CHR^3-CO-R^4$

$R^3$ einen aliphatischen, araliphatischen oder aromatischen Rest der Formel $-CR^7R^8R^9$ oder Phenyl, Pyridyl oder Thienyl, wobei die letztgenannten 3 Reste unsubstituiert oder durch 1 bis 3 Reste Z substituiert sind,

$R^7$ Wasserstoff, Halogen, $C_1-C_4$-Alkyl, Phenyl oder Benzyl, vorzugsweise Wasserstoff oder Methyl,

$R^8$, $R^9$ unabhängig voneinander Wasserstoff oder $C_1-C_4$-Alkyl oder $R^8$ und $R^9$ gemeinsam mit dem verbundenen C-Atom einen 5- oder 6-gliedrigen Ring und

Z unabhängig voneinander Halogen, $C_1-C_4$-Alkyl, $C_1-C_4$-Haloalkyl, $C_1-C_4$-Alkoxy, $C_1-C_4$-Haloalkoxy, $C_1-C_4$-Alkylthio, $C_1-C_4$-Alkylamino, Di-($C_1-C_4$-alkyl)-amino oder Nitro

bedeuten.

Vorzugsweise ist $R^3$ Isopropyl, 2,2-Dimethyl-ethyl oder Cyclopentyl.

Von besonderem Interesse sind auch erfindungsgemäße Verbindungen der Formel (I), worin

A einen Rest der Formel

$$R^5R^6C = \overset{|}{C} - CO - R^4$$

$R^5$ $C_1-C_4$-Alkyl, $C_2-C_4$-Alkenyl oder $C_2-C_4$-Alkinyl, Cyclohexyl, Cyclopentyl, Phenyl, Pyridyl oder Thienyl, wobei die letztgenannten 3 Reste unsubstituiert oder durch 1 bis 3 Reste Z substituiert sind,

$R^6$ Wasserstoff oder $C_1-C_4$-Alkyl und

Z unabhängig voneinander Halogen, $C_1-C_4$-Alkyl, $C_1-C_4$-Haloalkyl, $C_1-C_4$-Alkoxy, $C_1-C_4$-Haloalkoxy, $C_1-C_4$-Alkylthio, $C_1-C_4$-Alkylamino, Di-($C_1-C_4$-alkyl)-amino oder Nitro

bedeuten.

Von besonderem Interesse sind auch erfindungsgemäße Verbindungen der Formel (I), worin

$R^4$ einen $C_1-C_4$-Alkoxyrest, der durch $C_2-C_4$-Alkenyloxy, $C_2-C_4$-Alkinyloxy, Cyclohexyloxy, Cyclohexenyloxy, Cyclopentyloxy, Cyclopentenyloxy, Benzyloxy, Benzyl-

thio, $(R^*)_3Si$ oder $(R^*)_3SiO$, worin die Reste $R^*$ unabhängig voneinander für $C_1-C_4$-Alkyl, $C_2-C_4$-Alkenyl, $C_2-C_4$-Alkinyl oder Phenyl stehen, Cyano, Nitro, $(C_1-C_4$-Alkyl)-carbonyl, $C_1-C_4$-Alkylsulfonyl, $C_1-C_4$-Alkylsulfinyl, Di-$(C_1-C_4$-alkyl)-phosphinyl, Di-$(C_1-C_4$-alkyl)-phosphonyl, Di-$(C_1-C_4$-alkyl)-phosphoryl, Pyridyl substituiert ist, oder

$R^4$ den Rest eines 5- oder 6-Ringlactons, der die Bindung zum übrigen Molekülteil in $\alpha$-Position zur Carbonylgruppe hat, oder

$R^4$ einen Rest der Formel

$-D^7-CR^{15}R^{16}-D^8-L$ oder

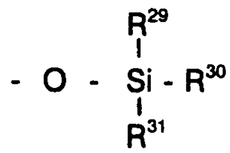

| | |
|---|---|
| $D^7$ | O, S, NH, Methylamino oder Ethylamino, |
| $D^8$ | eine direkte Bindung, |
| $R^{15}$, $R^{16}$ | unabhängig voneinander Wasserstoff, $C_1-C_4$-Alkyl, $C_2-C_4$-Alkenyl, $C_2-C_4$-Alkinyl, Phenyl oder Benzyl, vorzugsweise Wasserstoff oder Methyl, |
| L | einen Säurederivat-Rest der Formel |

$-CO-O-R^{17}$
$-CO-S-R^{18}$
$-CO-NR^{19}R^{20}$
$-CO-O-N=CR^{21}R^{22}$
$-CO-N=CR^{21}R^{22}$
$-O-CO-R^{23}$
$-S-CO-R^{23}$
$-NR^{14}-CO-R^{23}$

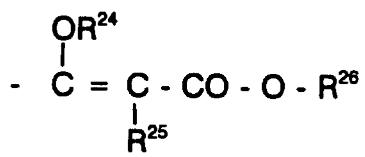 oder

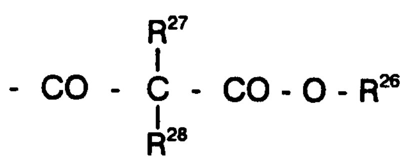

| | |
|---|---|
| $R^{17}$ | Wasserstoff, $C_2-C_4$-Alkenyl oder $C_2-C_4$-Alkinyl, Cyclohexyl, Cyclopentyl, Cyclohexenyl, Cyclopentenyl, Benzyl, Phenyl oder substituiertes Phenyl, |
| $R^{18}$ | Wasserstoff, $C_1-C_4$-Alkyl, $C_2-C_4$-Alkenyl oder $C_2-C_4$-Alkinyl, Cyclohexyl, Cyclopentyl, Cyclohexenyl, Cyclopentenyl, Benzyl, Phenyl oder substituiertes Phenyl, |
| $R^{19}$, $R^{20}$ | unabhängig voneinander Wasserstoff, $C_1-C_4$-Alkyl, $C_2-C_4$-Alkenyl, $C_2-C_4$-Alkinyl, Cyclohexyl, Cyclopentyl, Benzyl, Phenyl oder substituiertes Phenyl oder gemeinsam mit dem verbundenen N-Atom einen heterocyclischen 5- oder 6-gliedrigen Ring, der neben dem N-Atom noch ein O-Atom als Heteroatom enthalten kann, |
| $R^{21}$, $R^{22}$ | unabhängig voneinander $C_1-C_4$-Alkyl, $C_2-C_4$-Alkenyl oder $C_2-C_4$-Alkinyl oder gemeinsam mit dem verbundenen C-Atom einen carbocylischen 5- bis 6-gliedrigen Rest, |
| $R^{23}$ | Wasserstoff, $C_1-C_4$-Alkyl, Cyclohexyl, Cyclopentyl, Phenyl oder substituiertes Phenyl, |

| | |
|---|---|
| $R^{24}$ | $C_1-C_4$-Alkyl, $C_2-C_4$-Alkenyl oder $C_2-C_4$-Alkinyl, wobei die letztgenannten 3 Reste unsubstituiert oder durch einen oder mehrere Reste aus der Gruppe Halogen oder $C_1-C_4$-Alkoxy substituiert sind, oder $C_1-C_4$-Alkanoyl, ($C_1-C_4$-Alkoxy)-carbonyl, Benzyl, Benzoxycarbonyl, Phenyl oder Benzoyl, wobei die letztgenannten 4 Reste unsubstituiert oder durch $C_1-C_4$-Alkyl, $C_1-C_4$-Alkoxy, Halogen oder Nitro substituiert sind, |
| $R^{25}$ | Wasserstoff oder $C_1-C_4$-Alkyl, |
| $R^{26}$ | Wasserstoff, $C_1-C_4$-Alkyl, $C_2-C_4$-Alkenyl oder $C_2-C_4$-Alkinyl, wobei die letztgenannten 3 Reste unsubstituiert oder durch einen oder mehrere Reste aus der Gruppe Halogen substituiert sind, |
| $R^{27}$, $R^{28}$ | unabhängig voneinander Wasserstoff oder $C_1-C_4$-Alkyl und |
| $R^{29}$, $R^{30}$, $R^{31}$ | unabhängig voneinander $C_1-C_4$-Alkyl, $C_2-C_4$-Alkenyl oder $C_2-C_4$-Alkinyl, Phenyl oder substituiertes Phenyl |

bedeuten.

Bevorzugt sind erfindungsgemäße Verbindungen der Formel (I), worin

$R^4$ einen $C_1-C_4$-Alkoxyrest, der durch Allyl, Propargyl, $(R^*)_3Si$ oder $(R^*)_3SiO$, worin die Reste $R^*$ unabhängig voneinander für Methyl, Ethyl oder Phenyl stehen, oder Cyano, $C_1-C_4$-Alkylsulfonyl, $C_1-C_4$-Alkylsulfinyl, Di-($C_1-C_4$-alkyl)-phosphinyl, Di-($C_1-C_4$-alkyl)-phosphonyl, Di-($C_1-C_4$-alkyl)phosphoryl oder Pyridyl substituiert ist, oder

$R^4$ den Rest eines 5-Ringlactons, der die Bindung zum übrigen Molekülteil in $\alpha$-Position zur Carbonylgruppe hat, oder

$R^4$ einen Rest der Formel

$$-D^7-CR^{15}R^{16}-D^8-L \text{ oder}$$

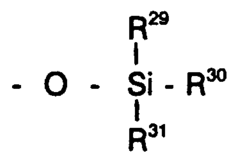

| | |
|---|---|
| $D^7$ | O, S oder NH, |
| $D^8$ | eine direkte Bindung, |
| $R^{15}$, $R^{16}$ | unabhängig voneinander Wasserstoff oder Methyl, |
| L | einen Säurederivat-Rest der Formel |

$-CO-O-R^{17}$

$-CO-S-R^{18}$

$-CO-N=CR^{21}R^{22}$

$-CO-O-N=CR^{21}R^{22}$

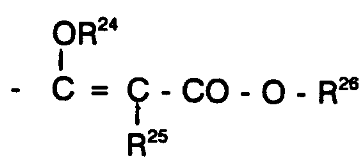 oder

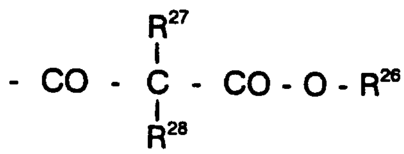

| | |
|---|---|
| $R^{17}$ | Wasserstoff, $C_2-C_4$-Alkenyl oder $C_2-C_4$-Alkinyl, Benzyl oder Phenyl, |
| $R^{18}$ | Wasserstoff, $C_1-C_4$-Alkyl, $C_2-C_4$-Alkenyl oder $C_2-C_4$-Alkinyl, Benzyl oder Phenyl, |

$R^{19}$, $R^{20}$ unabhängig voneinander Wasserstoff, $C_1-C_4$-Alkyl, $C_2-C_4$-Alkenyl, $C_2-C_4$-Alkinyl, Cyclohexyl, Cyclopentyl, Benzyl, Phenyl oder substituiertes Phenyl oder gemeinsam mit dem verbundenen N-Atom einen heterocyclischen 5- oder 6-gliedrigen Ring, der neben dem N-Atom noch ein O-Atom als Heteroatom enthalten kann,

$R^{24}$ $C_1-C_4$-Alkyl, wobei der letztgenannte Rest unsubstituiert oder durch einen oder mehrere Reste aus der Gruppe Halogen oder $C_1-C_4$-Alkoxy substituiert ist, oder $C_1-C_4$-Alkanoyl, ($C_1-C_4$-Alkoxy)-carbonyl, Benzyl, Benzoxycarbonyl, Phenyl oder Benzoyl, wobei die letztgenannten 4 Reste unsubstituiert oder durch $C_1-C_4$-Alkyl, $C_1-C_4$-Alkoxy, Halogen oder Nitro substituiert sind,

$R^{25}$ Wasserstoff oder $C_1-C_4$-Alkyl,

$R^{26}$ Wasserstoff, $C_1-C_4$-Alkyl, $C_2-C_4$-Alkenyl oder $C_2-C_4$-Alkinyl,

$R^{27}$, $R^{28}$ unabhängig voneinander Wasserstoff oder $C_1-C_4$-Alkyl und

$R^{29}$, $R^{30}$, $R^{31}$ unabhängig voneinander $C_1-C_4$-Alkyl, $C_2-C_4$-Alkenyl oder $C_2-C_4$-Alkinyl oder Phenyl

bedeuten.

Bevorzugt sind auch erfindungsgemäße Verbindungen der Formel (I), worin die einzelnen Reste und Gruppen zwei oder mehrere vorstehend als bevorzugt bezeichnete Reste oder Gruppen enthalten.

Ein weiterer Gegenstand der vorliegenden Erfindung ist das Verfahren zur Herstellung von Verbindungen der allgemeinen Formel I, dadurch gekennzeichnet, daß man

(a) falls A ein Rest der Formel $-CHR^3-CO-R^4$ ist, eine Verbindung der Formel (II)

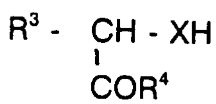 (II),

worin $R^3$, $R^4$ und X die bei Formel (I) genannten Bedeutungen haben, mit einer Verbindung der Formel (III)

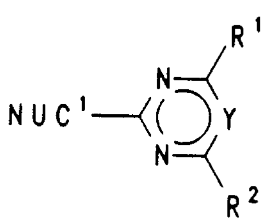 (III),

worin $NUC^1$ eine Abgangsgruppe bedeutet, beispielsweise aus der Gruppe Halogen, Alkylsulfonyl, Benzylsulfonyl und substituiertes Benzylsulfonyl, und $R^1$, $R^2$ und Y die bei Formel (I) genannte Bedeutung haben,
in Gegenwart einer anorganischen oder organischen Base umsetzt,
(b) falls A ein Rest der Formel $-CHR^3-CO-R^4$ ist, eine Verbindung der Formel (IV)

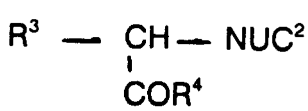 (IV),

worin $NUC^2$ eine Abgangsgruppe, beispielsweise aus der Gruppe Halogen und Methylsulfonyloxy, bedeutet und $R^3$ und $R^4$ wie bei Formel (I) definiert sind,
mit einer Verbindung der Formel (V)

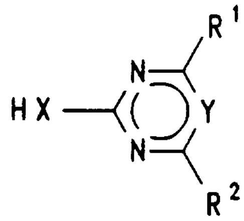

(V),

worin $R^1$, $R^2$, X und Y die bei Formel (I) genannten Bedeutungen haben, in Gegenwart einer anorganischen oder organischen Base umsetzt,

(c) falls A die Gruppe

$$R^5R^6C = C\text{-}CO\text{-}R^4$$

|

ist, eine Verbindung der Formel (VI)

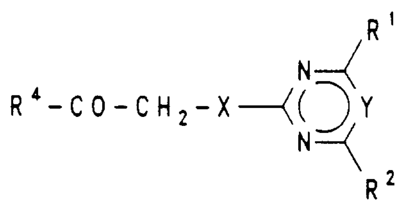

(VI),

worin

$R^1$, $R^2$, $R^4$, X und Y die bei Formel (I) genannten Bedeutungen haben, mit einem Aldehyd oder Keton der Formel (VII)

$R^5 - CO - R^6$ (VII),

worin $R^5$ und $R^6$ die bei Formel (I) genannten Bedeutungen haben, in Gegenwart einer geeigneten anorganischen oder organischen Base umsetzt,

(d) falls A ein Rest der Formel $-CHR^3-CO-R^4$ ist, eine Verbindung der unter (c) genannten Formel (VI) mit einer Halogenverbindung der Formel (VIII)

$R^3 - Hal$ (VIII),

worin Hal Chlor, Brom oder Iod bedeutet und $R^3$ wie bei Formel (I) definiert ist, in Gegenwart einer anorganischen oder organischen Base umsetzt oder

(e) eine Verbindung der Formel (IX) bzw. (X)

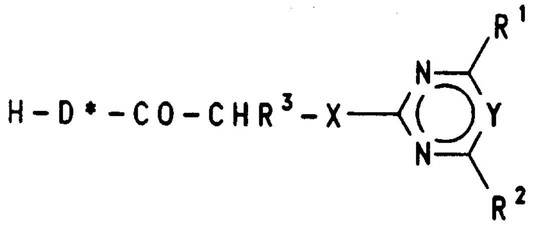

(IX)

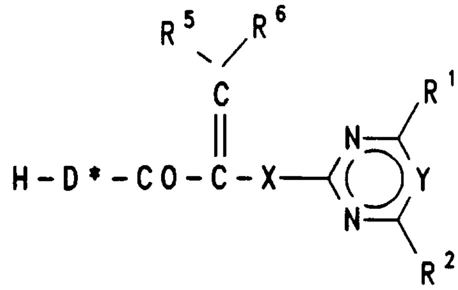

(X)

worin $R^1$, $R^2$, $R^3$, $R^5$, $R^6$, X und Y wie bei Formel (I) definiert sind und D* O, S oder $NR^{14}$ mit $R^{14}$ = H, Alkyl oder Phenyl bedeutet, mit einer Halogenverbindung der Formel (XI)

$R^0$ − Hal (XI)

worin $R^0$ so definiert ist, daß $R^0$−D*− die Bedeutung von $R^4$ nach Formel (I) hat in Gegenwart eine anorganischen oder organischen Base umsetzt oder
(f) eine Verbindung der Formel (IX) oder (X), in der D* ein Sauerstoffatom ist, mit Thionylchlorid, Oxalylchlorid, Chlorcarbonat, Carbonyldiimidazol oder Dicyclohexylcarbodiimid/4−N,N−Dimethylaminopyridin in an sich bekannter Weise zu einem aktivierten Carbonsäurederivat umsetzt und letzteres mit einer Verbindung der Formel (XII)

$R^4$ − H (XII)

in Gegenwart einer anorganischen oder organischen Base umsetzt.

Als Basen für die Umsetzungen (a) bis (e) sind beispielsweise anorganische Basen aus der Gruppe der Alkalicarbonate, wie $Na_2CO_3$, $K_2CO_3$, $NaHCO_3$ oder $KHCO_3$, Alkalihydride, wie NaH und KH, und Alkalifluoride, wie KF, oder organische Basen wie Triethylamin oder DBU (1,8−Diazabicyclo[5.4.0]undec−7−en) geeignet.

In der Regel ist es zweckmäßig, diese Umsetzungen in Gegenwart eines Lösungsmittels durchzuführen, z. B. mit einem Lösungsmittel wie Toluol, Xylol, Diethylethan, Diglyme, Monoglyme, Tetrahydrofuran, Dioxan, Dimethylformamid, N,N−Dimethylacetamid, Dimethylsulfoxid oder Acetonitril.

Auch kann es von Vorteil sein, die Umsetzungen in Gegenwart von Katalysatoren, beispielsweise von Kronenethern oder N,N,N',N'−Tetramethylendiamin, durchzuführen.

Als Basen für die Umsetzung (f) sind beispielsweise Alkalimetalle, wie Natrium oder Kalium, Alkalicarbonate, wie $K_2CO_3$, $Na_2CO_3$, $NaHCO_3$ oder $KHCO_3$, Alkali− und Erdalkalihydride, wie NaH, KH oder $CaH_2$, Alkalihydroxide wie KOH oder NaOH oder organische Basen wie Triethylamin geeignet. Als Lösungsmittel können bei dieser Umsetzung Kohlenwasserstoffe, wie n−Heptan, Benzol, Toluol oder Xylol, halogenierte Kohlenwasserstoffe, wie $CH_2Cl_2$, $CHCl_3$, $CCl_4$ und Chlorbenzol, Alkohole, wie $CH_3OH$, $C_2H_5OH$ oder 2−Propanol, Ether, wie Diethylether, Dioxan und Tetrahydrofuran (THF), Ketone, wie Aceton oder Methylethylketon, Ester, wie Ethylacetat, aprotisch polare Lösemittel, wie Dimethylformamid (DMF), N,N−Dimethylacetamid, Dimethylsulfoxid (DMSO), Acetonitril und Wasser, eingesetzt werden.

Die Ausgangssubstanzen der Formeln II−XII sind bekannt oder lassen sich analog bekannten Verfahren synthetisieren (vgl. Lit: EP−A−347811, EP−A−409369 (CA−A−2021486), EP−A−409368 (CA−A−2021486), EP−A−400741).

Die erfindungsgemäßen Verbindungen der Formel (I) weisen eine ausgezeichnete herbizide Wirksamkeit gegen ein breites Spektrum wirtschaftlich wichtiger mono−und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat−, Vorauflauf− oder Nachauflaufverfahren ausgebracht werden.

Im einzelnen seien beispielhaft einige Vertreter der mono− und dikotylen Unkrautflora genannt, die durch die erfindungsgemäßen Verbindungen kontrolliert werden können, ohne daß durch die Nennung eine Beschränkung auf bestimmte Arten erfolgen soll.

Auf der Seite der monokotylen Unkrautarten werden z.B. Avena, Lolium, Alopecurus, Phalaris, Echinochloa, Digitaria, Setaria sowie Cyperusarten aus der annuellen Gruppe und auf seiten der perennierenden Spezies Agropyron, Cynodon, Imperata sowie Sorghum und auch ausdauernde Cyperusarten gut erfaßt.

Bei dikotylen Unkrautarten erstreckt sich das Wirkungsspektrum auf Arten wie z.B. Galium, Viola, Veronica, Lamium, Stellaria, Amaranthus, Sinapis, Ipomoea, Matricaria, Abutilon und Sida auf der annuellen Seite sowie Convolvulus, Cirsium, Rumex und Artemisia bei den perennierenden Unkräutern.

Unter den spezifischen Kulturbedingungen im Reis vorkommende Unkräuter wie z.B. Sagittaria, Alisma, Eleocharis, Scirpus und Cyperus werden von den erfindungsgemäßen Wirkstoffen ebenfalls hervorragend bekämpft.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem

zum Applikationszeitpunkt vorhandenen Wachstumsstadium stehen oder sterben nach einer gewissen zeit ganz ab, so daß auf diese Weise eine für die Kulturpflanzen schädliche Unkrautkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Darüberhinaus weisen die erfindungsgemäßen Substanzen hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchstauchung eingesetzt werden. Desweiteren eignen sie sich auch zur generellen Steuerung und Hemmung von unenwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden.

Die Verbindungen der Formel (I) können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), emulgierbare Konzentrate (EC), konzentrierte Emulsionen (EW), z.B. Öl-in-Wasser- oder Wasser-in-Öl-Emulsionen, versprühbare Lösungen oder Emulsionen, Dispersionen auf Öl- oder Wasserbasis (SC), Stäubemittel (DP) Beizmittel, Granulate (G) wie Boden- bzw. Streugranulate (FG), wasserdispergierbare Granulate (WDG), ULV-Formulierungen, Mikrokapseln oder Wachse.

Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986; van Valkenburg, "Pesticides Formulations", Marcel Dekker N.Y., 2nd Ed. 1972-73; K. Martens, "Spray Drying Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J.; H.v. Olphen, "Introduction to Clay Colloid Chemistry"; 2nd Ed., J. Wiley & Sons, N.Y.; Marsden, "Solvents Guide", 2nd Ed., Interscience, N.Y. 1950; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Spritzpulver sind in Wasser gleichmäßig dispergierbare Präparate, die neben dem Wirkstoff und einem Verdünnungs- oder Inertstoff noch Netzmittel, z.B. polyoxyethylierte Alkylphenole, polyoxethylierte Fettalkohole, Alkan- oder Alkylarylsulfonate und Fettalkoholpolyglykolethersulfate, und Dispergiermittel, z.B. ligninsulfonsaures Natrium, 2,2'-dinaphthylmethan-6,6'-disulfonsaures Natrium, dibutylnaphthalin-sulfonsaures Natrium oder auch oleylmethyltaurinsaures Natrium enthalten.

Emulgierbare Konzentrate werden durch Auflösen des Wirkstoffes in einem organischen Lösungsmittel **z.B**. Cyclohexanon, Xylol oder auch höhersiedenden Aromaten oder Kohlenwasserstoffen unter Zusatz von einem oder mehreren Emulgatoren hergestellt. Als Emulgatoren können beispielsweise verwendet werden: Alkylarylsulfonsaure Calzium-Salze wie Ca-dodecylbenzolsulfonat, Fettalkoholpolyglykolethersulfate oder nichtionische Emulgatoren wie Fettsäurepolyglykolester, Alkylarylpolyglykolether, Fettalkoholpolyglykolether, Propylenoxid-Ethylenoxid-Kondensationsprodukte (z.B. Blockpolymere), Fettalkohol-Propylenoxid-Ethylenoxid-Kondensationsprodukte, Sorbitanfettsäureester, Polyoxyethylensorbitanfettsäureester oder Polyoxethylensorbitester.

Stäubemittel erhält man durch Vermahlen des Wirkstoffes mit fein verteilten festen Stoffen, z.B. Talkum, natürlichen Tonen, wie Kaolin, Bentonit und Pyrophillit, oder Diatomeenerde.

Granulate, wie Granulate zur Boden- bzw. Streuapplikation oder wasserdispergierbare Granulate, können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise, gewünschtenfalls in Mischung mit Düngemitteln, granuliert werden.

Teller-, Fließbett-, Extruder- und Sprühgranulate können nach üblichen Verfahren hergestellt werden; siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Informationen zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0,1 bis 99 Gewichtsprozent, insbesondere 0,1 bis 95 Gew.-%, Wirkstoff der Formel (I).

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 80, vorzugsweise 5 bis 80 Gew.-% betragen. Staubförmige Formulierungen enthalten 1 bis 30, vorzugsweise meistens 5 bis 20 Gew.-% an Wirkstoff, versprühbare Lösungen etwa 0,2 bis 25, vorzugsweise 2 bis 20 Gew.-% Wirkstoff. Bei wasserdispergierbren Granulaten hängt der Wirkstoffgehalt zum Teil davon ab, ob die wirksame Verbindung flüssig oder fest vorliegt und welche Granulierhilfsmittel, Füllstoffe usw. verwendet werden.

Im allgemeinen liegt der Gehalt bei den in Wasser dispergierbaren Granulaten zwischen 10 und 90 Gew.-%.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Auf der Basis dieser Formulierungen lassen sich auch Kombinationen mit anderen pestizid wirksamen Stoffen, Düngemitteln und/oder Wachstumsregulatoren herstellen, z.B. in Form einer Fertigformulierung oder als Tankmix. Als Kombinationspartner für die erfindungsgemäßen Wirkstoffe sind beispielsweise bekannte Wirkstoffe einsetzbar, wie sie in z.B. aus Weed Research 26, 441-445 (1986), oder "The Pesticide Manual", 9th edition, The British Crop Protection Council, 1990/91, Bracknell, England, und dort zitierter Literatur beschrieben sind. Als literaturbekannte Herbizide, die mit den Verbindungen der Formel (I) kombiniert werden können, sind z. B. folgende Wirkstoffe zu nennen (Anmerkung: Die Verbindungen sind entweder mit dem "common name" nach der International Organization for Standardization (ISO) oder mit dem chemischen Namen, ggf. zusammen mit einer üblichen Codenummer bezeichnet):
acetochlor; acifluorfen; aclonifen; AKH 7088, d. h. [[[1-[5-[2-Chloro-4-(trifluoromethyl)-phenoxy]-2-nitrophenyl]-2-methoxyethylidene]-amino]-oxy]-essigsäure und -essigsäuremethylester; alachlor; alloxydim; ametryn; amidosulfuron; amitrol; AMS, d. h. Ammoniumsulfamat; anilofos; asulam; atrazin; aziprotryn; barban; BAS 516 H, d. h. 5-Fluor-2-phenyl-4H-3,1-benzoxazin-4-on; benazolin; benfluralin; benfuresate; bensulfuron-methyl; bensulide; bentazone; benzofenap; benzofluor; benzoylprop-ethyl; benzthiazuron; bialaphos; bifenox; bromacil; bromobutide; bromofenoxim; bromoxynil; bromuron; buminafos; busoxinone; butachlor; butamifos; butenachlor; buthidazole; butralin; butylate; carbetamide; CDAA, d. h. 2-Chlor-N,N-di-2-propenylacetamid; CDEC, d. h. Diethyldithiocarbaminsäure-2-chlorallylester; CGA 184927, d. h. 2-[4-[(5-Chlor-3-fluor-2-pyridinyl)-oxy]-phenoxy]-propansaure und 2-propynylester; chlomethoxyfen; chloramben; chlorazifop-butyl, pirifenop-butyl; chlorbromuron; chlorbufam; chlorfenac; chlorflurecol-methyl; chloridazon; chlorimuron ethyl; chlornitrofen; chlorotoluron; chloroxuron; chlorpropham; chlorsulfuron; chlorthal-dimethyl; chlorthiamid; cinmethylin; cinosulfuron; clethodim; clomazone; clomeprop; cloproxydim; clopyralid; cyanazine; cycloate; cycloxydim; cycluron; cyperquat; cyprazine; cyprazole; 2,4-DB; dalapon; desmediphan; desmetryn; di-allate; dicamba; dichlobenil; dichlorprop; diclofop-methyl; diethatyl; difenoxuron; difenzoquat; diflufenican; dimefuron; dimethachlor; dimethametryn; dimethazone, clomazon; dimethipin; dimetrasulfuron, cinosulfuron; dinitramine; dinoseb; dinoterb; diphenamid; dipropetryn; diquat; dithiopyr; diuron; DNOC; eglinazine-ethyl; EL 177, d. h. 5-Cyano-1-(1,1-dimethylethyl)-N-methyl-3H-pyrazole-4-carboxamid; endothal; EPTC; esprocarb; ethalfluralin; ethametsulfuron-methyl; ethidimuron; ethiozin; ethofumesate; F5231, d. h. N-[2-Chlor-4-fluor-5-[4-(3-fluorpropyl)-4,5-dihydro-5-oxo-1H-tetrazol-1-yl]-phenyl]-ethansulfonamid; F6285, d. h. 1-[5-(N-Methylsulfonyl)-amino-2,4-dichlorophenyl]-3-methyl-4-difluoromethyl-1,2,4-triazol-5-on; fenoprop; fenoxan, s. clomazon; fenoxaprop-ethyl; fenuron; flamprop-methyl; flazasulfuron; fluazifop und dessen Esterderivate; fluchloralin; flumetsulam; N-[2,6-Difluorphenyl]-5-methyl-(1,2,4)-triazolo[1,5a]pyrimidin-2-sulfonamid; flumeturon; flumipropyn; fluorodifen; fluoroglycofen-ethyl; fluridone; flurochloridone; fluroxypry; flurtamone; fomesafen; fosamine; furyloxyfen; glufosinate; glyphosate; halosafen; haloxyfop und dessen Esterderivate; hexazinone; Hw 52, d. h. N-(2,3-Dichlorphenyl)-4-(ethoxymethoxy)-benzamid; imazamethabenz-methyl; imazapyr; imazaquin; imazethamethapyr; imazethapyr; imazosulfuron; ioxynil; isocarbamid; isopropalin; isoproturon; isouron; isoxaben; isoxapyrifop; karbutilate; lactofen; lenacil; linuron; MCPA; MCPB; mecoprop; mefenacet; mefluidid; metamitrol; metazachlor; methabenzthiazuron; metham; methazole; methoxyphenone; methyldymron; metobro-muron; metolachlor; metoxuron; metribuzin; metsulfuron-methyl; MH; molinate; monalide; monocarbamide dihydrogensulfate; monolinuron; monuron; MT 128, d. h. 6-Chlor-N-(3-chlor-2-propenyl)-5-methyl-N-phenyl-3-pyridazinamin; MT 5950, d. h. N-[3-Chlor-4-(1-methylethyl)-phenyl]-2-methylpentanamid; naproanilide; napropamide; naptalam; NC 310, d. h. 4-(2,4-dichlorbenzoyl)-1-methyl-5-benzyloxypyrazol; neburon; nicosulfuron; nipyraclophen; nitralin; nitrofen; nitrofluorfen; norflura-zon; orbencarb; oryzalin; oxadiazon; oxyfluorfen; paraquat; pebulate; pendimethalin; perfluidone; phenmedi-pham; phenisopham; phenmedipham; picloram; piperophos; piributicarb; pirifenop-butyl; pretilachlor; primisulfuron-methyl; procyazine; prodiamine; profluralin; proglinazine-ethyl; prometon; prometryn; pro-pachlor; propanil; propaquizafop und dessen Esterderivate; propazine; propham; propyzamide; prosulfalin; prosulfocarb; prynachlor; pyrazolinate; pyrazon; pyrazosulfuron-ethyl; pyrazoxyfen; pyridate; quinclorac; quinmerac; quinofop und dessen Esterderivate, quizalofop und dessen Esterderivate; quizalofop-ethyl; quizalofop-p-tefuryl; renriduron; dymron; S 275, d. h. 2-[4-Chlor-2-fluor-5-(2-propynyloxy)-phenyl]-4,5,6,7-tetrahydro-2H-indazol; S 482, d. h. 2-[7-Fluor-3,4-dihydro-3-oxo-4-(2-pro-pynyl)-2H-1,4-benzoxazin-6-yl]-4,5,6,7-tetrahydro-1H-isoindol-1,3(2H)-dion; secbumeton; sethoxydim; siduron; simazine; simetryn; SN 106279, d. h. 2-[[7-[2-Chlor-4-(trifluor-methyl)-phe-noxy]-2-naphthalenyl]-oxy]propansäure und -methylester; sulfometuron-methyl; sulfazuron; flazasul-furon; TCA; tebutam; tebuthiuron; terbacil; terbucarb; terbuchlor; terbumeton; terbuthylazine; terbutryn; TFH 450, d. h. N,N-Diethyl-3-[(2-ethyl-6 methylphenyl)-sulfonyl]-1H-1,2,4-triazol-1-carboxamid; thiazafluron; thifensulfuron-methyl; thiobencarb; tiocarbazil; tralkoxydim; tri-allate; triasulfuron; triazofena-mide; tribenuron-methyl; triclopyr; tridiphane; trietazine; trifluralin; trimeturon; vernolate; WL 110547, d. h. 5-Phenoxy-1-[3-(trifluormethyl)-phenyl]-1H-tetrazol.

Zur Anwendung werden die in handelsüblicher Form vorliegenden Formulierungen gegebenenfalls in üblicher Weise verdünnt, z.B. bei Spritzpulvern, emulgierbaren Konzentraten, Dispersionen und wasserdis-pergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,005 und 10,0 kg/ha oder mehr Aktivsubstanz, vorzugsweise liegt sie jedoch zwischen 0,01 und 5 kg/ha.

Formulierungsbeispiele

a) Ein Stäubemittel wird erhalten, indem man 10 Gew.-Teile einer Verbindung der Formel (I) und 90 Gew.-Teile Talkum als Inertstoff mischt und in einer Schlagmühle zerkleinert.

b) Ein in Wasser leicht dispergierbares, benetzbares Pulver wird erhalten, indem man 25 Gewichtsteile einer Verbindung der Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.

c) Ein in Wasser leicht dispergierbares Dispersionskonzentrat wird erhalten, indem man 20 Gewichtsteile einer Verbindung der Formel (I) mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277°C) mischt und in einer Reibkugelmühle auf eine Feinheit von unter 5 Mikron vermahlt.

d) Ein emulgierbares Konzentrat wird erhalten aus 15 Gew.-Teilen einer Verbindung der Formel (I), 75 Gew.-Teilen Cyclohexan als Lösungsmittel und 10 Gew.-Teilen oxethyliertes Nonylphenol als Emul-gator.

e) Ein in Wasser dispergierbares Granulat wird erhalten indem man

| | |
|---|---|
| 75 Gewichtsteile | einer Verbindung der Formel (I), |
| 10 " | ligninsulfonsaures Calcium, |
| 5 " | Natriumlaurylsulfat, |
| 3 " | Polyvinylalkohol und |
| 7 " | Kaolin |

mischt, auf einer Stiftmühle mahlt und das Pulver in einem Wirbelbett durch Aufsprühen von Wasser als Granulierflüssigkeit granuliert.

f) Ein in Wasser dispergierbares Granulat wird auch erhalten, indem man

| | |
|---|---|
| 25 Gewichtsteile | einer Verbindung der Formel (I), |
| 5 " | 2,2' - dinaphthylmethan - 6,6' - disulfonsaures Natrium |
| 2 " | oleoylmethyltaurinsaures Natrium, |
| 1 Gewichtsteil | Polyvinylalkohol, |
| 17 Gewichtsteile | Calciumcarbonat und |
| 50 " | Wasser |

auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

Chemische Beispiele

2 - (4,6 - Dimethoxypyrimidinyl - 2 - oxy) - 3 - methylbutansäure - (benzyloxycarbonyl) - methylester (Bsp. 61)

Zu einer Lösung von 4 g (15,6 mMol) 2 - (4,6 - Dimethoxypyrimidinyl - 2 - oxy) - 3 - methylbutansäure und 3,58 g (15,6 mMol) Bromessigsäurebenzylester in 25 ml Dimethylformamid gibt man unter Rühren bei Raumtemperatur 0,91 g (15,6 mMol) Kaliumfluorid. Nach 10 h Rühren bei 90 °C gibt man die Reaktionslösung auf Eiswasser und extrahiert mit Ethylacetat. Einrotieren liefert 5,19 g der Titelverbindung (Bsp 61) in Form eines farblosen Öls.

2 - (4,6 - Dimethoxypyrimidinyl - 2 - oxy) - 3 - methylbutansäure - (trimethylsilyl) - methylester (Bsp. 391)

Zu einer Lösung von 3 g (11,7 mMol) 2 - (4,6 - Dimethoxypyrimidinyl - 2 - oxy) - 3 - methylbutansäure, 1,22 g (11,7 mMol) Trimethylsilylmethanol und 0,05 g 4 - Dimethylaminopyridin in 25 ml $CH_2Cl_2$ tropft man unter Rühren bei 20 °C 2,41 g (11,7 mMol) Dicyclohexylcarbodiimid zu. Nach 12 h Rühren bei Raumtemperatur und anschließender Säulenfiltration erhält man 2,9 g der Titelverbindung (Bsp. 391) in Form eines farblosen Öls.

Die übrigen Verbindungen in der nachfolgenden Tabelle I werden analog den vorstehend beschriebenen Beispielen und Verfahrensvarianten erhalten.

Tabelle I: Verbindungen der Formel Ia

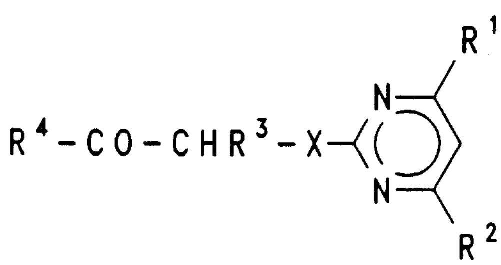

(Ia)

Anmerkung: In der Tabelle I bedeutet Ph = Phenyl, Pr = Propyl und Bu = Butyl.

| Bsp-Nr. | $R^1$ | $R^2$ | X | Y | $R^3$ | $R^4$ | Schmp.(C°) bzw.$[n]_D^{20}$ |
|---|---|---|---|---|---|---|---|
| 1 | $OCH_3$ | $OCH_3$ | O | CH | i-$C_3H_7$ | $OCH_2COOCH_2CH=CH_2$ | 52 |
| 2 | Cl | " | " | " | " | " | |
| 3 | $CH_3$ | " | " | " | " | " | |
| 4 | $NHCH_3$ | " | " | " | " | " | |
| 5 | $OCH_3$ | " | S | " | " | " | |
| 6 | Cl | " | " | " | " | " | |
| 7 | $CH_3$ | " | " | " | " | " | |
| 8 | $NHCH_3$ | " | " | " | " | " | |
| 9 | $OCH_3$ | " | " | N | " | " | |
| 10 | " | " | O | " | " | " | |
| 11 | " | " | " | CH | t-$C_4H_9$ | " | |
| 12 | Cl | " | " | " | " | " | |
| 13 | $CH_3$ | " | " | " | " | " | |
| 14 | $NHCH_3$ | " | " | " | " | " | |
| 15 | $OCH_3$ | " | " | " | " | " | |
| 16 | Cl | " | " | " | " | " | |
| 17 | $CH_3$ | " | " | " | " | " | |
| 18 | $NHCH_3$ | " | " | " | " | " | |
| 19 | $OCH_3$ | " | " | N | " | " | |
| 20 | " | " | O | " | " | " | |
| 21 | " | " | " | CH | Cyclopentyl | " | 56-57 |
| 22 | Cl | " | " | " | " | " | |
| 23 | $CH_3$ | " | " | " | " | " | |
| 24 | $NHCH_3$ | " | " | " | " | " | |
| 25 | $OCH_3$ | " | S | " | " | " | |
| 26 | Cl | " | " | " | " | " | |
| 27 | $CH_3$ | " | " | " | " | " | |
| 28 | $NHCH_3$ | " | " | " | " | " | |
| 29 | $OCH_3$ | " | " | N | " | " | |
| 30 | " | " | O | " | " | " | |

| Bsp-Nr. | $R^1$ | $R^2$ | X | Y | $R^3$ | $R^4$ | Schmp.(C°) bzw.$[n]_D^{20}$ |
|---|---|---|---|---|---|---|---|
| 31 | $OCH_3$ | $OCH_3$ | O | CH | i-$C_3H_7$ | $OCH_2COOCH_2C{\equiv}CH$ | 98 |
| 32 | Cl | " | " | " | " | " | |
| 33 | $CH_3$ | " | " | " | " | " | |
| 34 | $NHCH_3$ | " | " | " | " | " | |
| 35 | $OCH_3$ | " | S | " | " | " | |
| 36 | Cl | " | " | " | " | " | |
| 37 | $CH_3$ | " | " | " | " | " | |
| 38 | $NHCH_3$ | " | " | " | " | " | |
| 39 | $OCH_3$ | " | " | N | " | " | |
| 40 | " | " | O | " | " | " | |
| 41 | " | " | " | " | t-$C_4H_9$ | " | |
| 42 | Cl | " | " | " | " | " | |
| 43 | $CH_3$ | " | " | " | " | " | |
| 44 | $NHCH_3$ | " | " | " | " | " | |
| 45 | $OCH_3$ | " | " | " | " | " | |
| 46 | Cl | " | " | " | " | " | |
| 47 | $CH_3$ | " | " | " | " | " | |
| 48 | $NHCH_3$ | " | " | " | " | " | |
| 49 | $OCH_3$ | " | " | N | " | " | |
| 50 | " | " | O | " | " | " | |
| 51 | " | " | " | CH | Cyclopentyl | " | 81-82 |
| 52 | Cl | " | " | " | " | " | |
| 53 | $CH_3$ | " | " | " | " | " | |
| 54 | $NHCH_3$ | " | " | " | " | " | |
| 55 | $OCH_3$ | " | S | " | " | " | |
| 56 | Cl | " | " | " | " | " | |
| 57 | $CH_3$ | " | " | " | " | " | |
| 58 | $NHCH_3$ | " | " | " | " | " | |
| 59 | $OCH_3$ | " | " | N | " | " | |
| 60 | " | " | O | " | " | " | |

| Bsp-Nr. | $R^1$ | $R^2$ | X | Y | $R^3$ | $R^4$ | Schmp.(C°) bzw.$[n]_D^{20}$ |
|---|---|---|---|---|---|---|---|
| 61 | $OCH_3$ | $OCH_3$ | O | CH | $i-C_3H_7$ | $OCH_2COOCH_2Ph$ | Öl |
| 62 | Cl | " | " | " | " | " | |
| 63 | $CH_3$ | " | " | " | " | " | |
| 64 | $NHCH_3$ | " | " | " | " | " | |
| 65 | $OCH_3$ | " | S | " | " | " | |
| 66 | Cl | " | " | " | " | " | |
| 67 | $CH_3$ | " | " | " | " | " | |
| 68 | $NHCH_3$ | " | " | " | " | " | |
| 69 | $OCH_3$ | " | " | N | " | " | |
| 70 | " | " | O | " | " | " | |
| 71 | " | " | " | " | t-Bu | " | |
| 72 | Cl | " | " | " | " | " | |
| 73 | $CH_3$ | " | " | " | " | " | |
| 74 | $NHCH_3$ | " | " | " | " | " | |
| 75 | $OCH_3$ | " | " | " | " | " | |
| 76 | Cl | " | " | " | " | " | |
| 77 | $CH_3$ | " | " | " | " | " | |
| 78 | $NHCH_3$ | " | " | " | " | " | |
| 79 | $OCH_3$ | " | " | N | " | " | |
| 80 | " | " | O | " | " | " | |
| 81 | " | " | " | CH | Cyclopentyl | " | 66-68 |
| 82 | Cl | " | " | " | " | " | |
| 83 | $CH_3$ | " | " | " | " | " | |
| 84 | $NHCH_3$ | " | " | " | " | " | |
| 85 | $OCH_3$ | " | S | " | " | " | |
| 86 | Cl | " | " | " | " | " | |
| 87 | $CH_3$ | " | " | " | " | " | |
| 88 | $NHCH_3$ | " | " | " | " | " | |
| 89 | $OCH_3$ | " | " | N | " | " | |
| 90 | " | " | O | " | " | " | |

| Bsp-Nr. | $R^1$ | $R^2$ | X | Y | $R^3$ | $R^4$ | Schmp.(C°) bzw. $[n]_D^{20}$ |
|---|---|---|---|---|---|---|---|
| 91 | $OCH_3$ | $OCH_3$ | O | CH | $i\text{-}C_3H_7$ | $OCH_2COSCH_3$ | |
| 92 | Cl | " | " | " | " | " | |
| 93 | $CH_3$ | " | " | " | " | " | |
| 94 | $NHCH_3$ | " | " | " | " | " | |
| 95 | $OCH_3$ | " | S | " | " | " | |
| 96 | Cl | " | " | " | " | " | |
| 97 | $CH_3$ | " | " | " | " | " | |
| 98 | $NHCH_3$ | " | " | " | " | " | |
| 99 | $OCH_3$ | " | " | N | " | " | |
| 100 | " | " | O | " | " | " | |
| 101 | " | " | " | " | t-Bu | " | |
| 102 | Cl | " | " | " | " | " | |
| 103 | $CH_3$ | " | " | " | " | " | |
| 104 | $NHCH_3$ | " | " | " | " | " | |
| 105 | $OCH_3$ | " | " | " | " | " | |
| 106 | Cl | " | " | " | " | " | |
| 107 | $CH_3$ | " | " | " | " | " | |
| 108 | $NHCH_3$ | " | " | " | " | " | |
| 109 | $OCH_3$ | " | " | N | " | " | |
| 110 | " | " | O | " | " | " | |
| 111 | " | " | " | CH | Cyclopentyl | " | |
| 112 | Cl | " | " | " | " | " | |
| 113 | $CH_3$ | " | " | " | " | " | |
| 114 | $NHCH_3$ | " | " | " | " | " | |
| 115 | $OCH_3$ | " | S | " | " | " | |
| 116 | Cl | " | " | " | " | " | |
| 117 | $CH_3$ | " | " | " | " | " | |
| 118 | $NHCH_3$ | " | " | " | " | " | |
| 119 | $OCH_3$ | " | " | N | " | " | |
| 120 | " | " | O | " | " | " | |

| Bsp-Nr. | $R^1$ | $R^2$ | X | Y | $R^3$ | $R^4$ | Schmp.(C°) bzw.$[n]_D^{20}$ |
|---|---|---|---|---|---|---|---|
| 121 | $OCH_3$ | $OCH_3$ | O | CH | i-$C_3H_7$ | $OCH_2COSC_2H_5$ | |
| 122 | Cl | " | " | " | " | " | |
| 123 | $CH_3$ | " | " | " | " | " | |
| 124 | $NHCH_3$ | " | " | " | " | " | |
| 125 | $OCH_3$ | " | S | " | " | " | |
| 126 | Cl | " | " | " | " | " | |
| 127 | $CH_3$ | " | " | " | " | " | |
| 128 | $NHCH_3$ | " | " | " | " | " | |
| 129 | $OCH_3$ | " | " | N | " | " | |
| 130 | " | " | O | " | " | " | |
| 131 | " | " | " | " | t-Bu | " | |
| 132 | Cl | " | " | " | " | " | |
| 133 | $CH_3$ | " | " | " | " | " | |
| 134 | $NHCH_3$ | " | " | " | " | " | |
| 135 | $OCH_3$ | " | " | " | " | " | |
| 136 | Cl | " | " | " | " | " | |
| 137 | $CH_3$ | " | " | " | " | " | |
| 138 | $NHCH_3$ | " | " | " | " | " | |
| 139 | $OCH_3$ | " | " | N | " | " | |
| 140 | " | " | O | " | " | " | |
| 141 | " | " | " | CH | Cyclopentyl | " | |
| 142 | Cl | " | " | " | " | " | |
| 143 | $CH_3$ | " | " | " | " | " | |
| 144 | $NHCH_3$ | " | " | " | " | " | |
| 145 | $OCH_3$ | " | S | " | " | " | |
| 146 | Cl | " | " | " | " | " | |
| 147 | $CH_3$ | " | " | " | " | " | |
| 148 | $NHCH_3$ | " | " | " | " | " | |
| 149 | $OCH_3$ | " | " | N | " | " | |
| 150 | " | " | O | " | " | " | |

| Bsp-Nr. | $R^1$ | $R^2$ | X | Y | $R^3$ | $R^4$ | Schmp.(C°) bzw.$[n]_D^{20}$ |
|---|---|---|---|---|---|---|---|
| 151 | $OCH_3$ | $OCH_3$ | O | CH | i-$C_3H_7$ | $OCH_2COSCH_2Ph$ | |
| 152 | Cl | " | " | " | " | " | |
| 153 | $CH_3$ | " | " | " | " | " | |
| 154 | $NHCH_3$ | " | " | " | " | " | |
| 155 | $OCH_3$ | " | S | " | " | " | |
| 156 | Cl | " | " | " | " | " | |
| 157 | $CH_3$ | " | " | " | " | " | |
| 158 | $NHCH_3$ | " | " | " | " | " | |
| 159 | $OCH_3$ | " | " | N | " | " | |
| 160 | " | " | O | " | " | " | |
| 161 | " | " | " | " | t-Bu | " | |
| 162 | Cl | " | " | " | " | " | |
| 163 | $CH_3$ | " | " | " | " | " | |
| 164 | $NHCH_3$ | " | " | " | " | " | |
| 165 | $OCH_3$ | " | " | " | " | " | |
| 166 | Cl | " | " | " | " | " | |
| 167 | $CH_3$ | " | " | " | " | " | |
| 168 | $NHCH_3$ | " | " | " | " | " | |
| 169 | $OCH_3$ | " | " | N | " | " | |
| 170 | " | " | O | " | " | " | |
| 171 | " | " | " | CH | Cyclopentyl | " | |
| 172 | Cl | " | " | " | " | " | |
| 173 | $CH_3$ | " | " | " | " | " | |
| 174 | $NHCH_3$ | " | " | " | " | " | |
| 175 | $OCH_3$ | " | S | " | " | " | |
| 176 | Cl | " | " | " | " | " | |
| 177 | $CH_3$ | " | " | " | " | " | |
| 178 | $NHCH_3$ | " | " | " | " | " | |
| 179 | $OCH_3$ | " | " | N | " | " | |
| 180 | " | " | O | " | " | " | |

| Bsp-Nr. | $R^1$ | $R^2$ | X | Y | $R^3$ | $R^4$ | Schmp.(C°) bzw.$[n]_D^{20}$ |
|---|---|---|---|---|---|---|---|
| 181 | $OCH_3$ | $OCH_3$ | O | CH | i-$C_3H_7$ | $OCH(CH_3)COOCH_2CH=CH_2$ | |
| 182 | Cl | " | " | " | " | " | |
| 183 | $CH_3$ | " | " | " | " | " | |
| 184 | $NHCH_3$ | " | " | " | " | " | |
| 185 | $OCH_3$ | " | S | " | " | " | |
| 186 | Cl | " | " | " | " | " | |
| 187 | $CH_3$ | " | " | " | " | " | |
| 188 | $NHCH_3$ | " | " | " | " | " | |
| 189 | $OCH_3$ | " | " | N | " | " | |
| 190 | " | " | O | " | " | " | |
| 191 | " | " | " | " | t-$C_4H_9$ | " | |
| 192 | Cl | " | " | " | " | " | |
| 193 | $CH_3$ | " | " | " | " | " | |
| 194 | $NHCH_3$ | " | " | " | " | " | |
| 195 | $OCH_3$ | " | " | " | " | " | |
| 196 | Cl | " | " | " | " | " | |
| 197 | $CH_3$ | " | " | " | " | " | |
| 198 | $NHCH_3$ | " | " | " | " | " | |
| 199 | $OCH_3$ | " | " | N | " | " | |
| 200 | " | " | O | " | " | " | |
| 201 | " | " | " | CH | Cylopentyl | " | 62-64 |
| 202 | Cl | " | " | " | " | " | |
| 203 | $CH_3$ | " | " | " | " | " | |
| 204 | $NHCH_3$ | " | " | " | " | " | |
| 205 | $OCH_3$ | " | S | " | " | " | |
| 206 | Cl | " | " | " | " | " | |
| 207 | $CH_3$ | " | " | " | " | " | |
| 208 | $NHCH_3$ | " | " | " | " | " | |
| 209 | $OCH_3$ | " | " | N | " | " | |
| 210 | " | " | O | " | " | " | |

| Bsp-Nr. | $R^1$ | $R^2$ | X | Y | $R^3$ | $R^4$ | Schmp.(C°) bzw.$[n]_D^{20}$ |
|---|---|---|---|---|---|---|---|
| 211 | $OCH_3$ | $OCH_3$ | O | CH | i-$C_3H_7$ | $OCH(CH_3)COOCH_2C{\equiv}CH$ | 85 |
| 212 | Cl | " | " | " | " | " | |
| 213 | $CH_3$ | " | " | " | " | " | |
| 214 | $NHCH_3$ | " | " | " | " | " | |
| 215 | $OCH_3$ | " | S | " | " | " | |
| 216 | Cl | " | " | " | " | " | |
| 217 | $CH_3$ | " | " | " | " | " | |
| 218 | $NHCH_3$ | " | " | " | " | " | |
| 219 | $OCH_3$ | " | " | N | " | " | |
| 220 | " | " | O | " | " | " | |
| 221 | " | " | " | " | t-Bu | " | |
| 222 | Cl | " | " | " | " | " | |
| 223 | $CH_3$ | " | " | " | " | " | |
| 224 | $NHCH_3$ | " | " | " | " | " | |
| 225 | $OCH_3$ | " | " | " | " | " | |
| 226 | Cl | " | " | " | " | " | |
| 227 | $CH_3$ | " | " | " | " | " | |
| 228 | $NHCH_3$ | " | " | " | " | " | |
| 229 | $OCH_3$ | " | " | N | " | " | |
| 230 | " | " | O | " | " | " | |
| 231 | " | " | " | CH | Cyclopentyl | " | Öl |
| 232 | Cl | " | " | " | " | " | |
| 233 | $CH_3$ | " | " | " | " | " | |
| 234 | $NHCH_3$ | " | " | " | " | " | |
| 235 | $OCH_3$ | " | S | " | " | " | |
| 236 | Cl | " | " | " | " | " | |
| 237 | $CH_3$ | " | " | " | " | " | |
| 238 | $NHCH_3$ | " | " | " | " | " | |
| 239 | $OCH_3$ | " | " | N | " | " | |
| 240 | " | " | O | " | " | " | |

| Bsp-Nr. | $R^1$ | $R^2$ | X | Y | $R^3$ | $R^4$ | Schmp.(C°) bzw. $[n]_D^{20}$ |
|---|---|---|---|---|---|---|---|
| 241 | $OCH_3$ | $OCH_3$ | O | CH | $i\text{-}C_3H_7$ | $OCH(CH_3)COOCH_2Ph$ | |
| 242 | Cl | " | " | " | " | " | |
| 243 | $CH_3$ | " | " | " | " | " | |
| 244 | $NHCH_3$ | " | " | " | " | " | |
| 245 | $OCH_3$ | " | S | " | " | " | |
| 246 | Cl | " | " | " | " | " | |
| 247 | $CH_3$ | " | " | " | " | " | |
| 248 | $NHCH_3$ | " | " | " | " | " | |
| 249 | $OCH_3$ | " | " | N | " | " | |
| 250 | " | " | O | " | " | " | |
| 251 | " | " | " | " | t-Bu | " | |
| 252 | Cl | " | " | " | " | " | |
| 253 | $CH_3$ | " | " | " | " | " | |
| 254 | $NHCH_3$ | " | " | " | " | " | |
| 255 | $OCH_3$ | " | " | " | " | " | |
| 256 | Cl | " | " | " | " | " | |
| 257 | $CH_3$ | " | " | " | " | " | |
| 258 | $NHCH_3$ | " | " | " | " | " | |
| 259 | $OCH_3$ | " | " | N | " | " | |
| 260 | " | " | O | " | " | " | |
| 261 | " | " | " | CH | Cyclopentyl | " | |
| 262 | Cl | " | " | " | " | " | |
| 263 | $CH_3$ | " | " | " | " | " | |
| 264 | $NHCH_3$ | " | " | " | " | " | |
| 265 | $OCH_3$ | " | S | " | " | " | |
| 266 | Cl | " | " | " | " | " | |
| 267 | $CH_3$ | " | " | " | " | " | |
| 268 | $NHCH_3$ | " | " | " | " | " | |
| 269 | $OCH_3$ | " | " | N | " | " | |
| 270 | " | " | O | " | " | " | |

| Bsp-Nr. | $R^1$ | $R^2$ | X | Y | $R^3$ | $R^4$ | Schmp.(C °) bzw.$[n]_D^{20}$ |
|---|---|---|---|---|---|---|---|
| 271 | $OCH_3$ | $OCH_3$ | O | CH | i-$C_3H_7$ | $OCH(CH_3)COSCH_3$ | |
| 272 | Cl | " | " | " | " | " | |
| 273 | $CH_3$ | " | " | " | " | " | |
| 274 | $NHCH_3$ | " | " | " | " | " | |
| 275 | $OCH_3$ | " | S | " | " | " | |
| 276 | Cl | " | " | " | " | " | |
| 277 | $CH_3$ | " | " | " | " | " | |
| 278 | $NHCH_3$ | " | " | " | " | " | |
| 279 | $OCH_3$ | " | " | N | " | " | |
| 280 | " | " | O | " | " | " | |
| 281 | " | " | " | " | t-Bu | " | |
| 282 | Cl | " | " | " | " | " | |
| 283 | $CH_3$ | " | " | " | " | " | |
| 284 | $NHCH_3$ | " | " | " | " | " | |
| 285 | $OCH_3$ | " | " | " | " | " | |
| 286 | Cl | " | " | " | " | " | |
| 287 | $CH_3$ | " | " | " | " | " | |
| 288 | $NHCH_3$ | " | " | " | " | " | |
| 289 | $OCH_3$ | " | " | N | " | " | |
| 290 | " | " | O | " | " | " | |
| 291 | " | " | " | CH | Cyclopentyl | " | |
| 292 | Cl | " | " | " | " | " | |
| 293 | $CH_3$ | " | " | " | " | " | |
| 294 | $NHCH_3$ | " | " | " | " | " | |
| 295 | $OCH_3$ | " | S | " | " | " | |
| 296 | Cl | " | " | " | " | " | |
| 297 | $CH_3$ | " | " | " | " | " | |
| 298 | $NHCH_3$ | " | " | " | " | " | |
| 299 | $OCH_3$ | " | " | N | " | " | |
| 300 | " | " | O | " | " | " | |

| Bsp-Nr. | $R^1$ | $R^2$ | X | Y | $R^3$ | $R^4$ | Schmp.(C°) bzw.$[n]_D^{20}$ |
|---|---|---|---|---|---|---|---|
| 301 | $OCH_3$ | $OCH_3$ | O | CH | i-$C_3H_7$ | $OCH(CH_3)COSC_2H_5$ | |
| 302 | Cl | " | " | " | " | " | |
| 303 | $CH_3$ | " | " | " | " | " | |
| 304 | $NHCH_3$ | " | " | " | " | " | |
| 305 | $OCH_3$ | " | S | " | " | " | |
| 306 | Cl | " | " | " | " | " | |
| 307 | $CH_3$ | " | " | " | " | " | |
| 308 | $NHCH_3$ | " | " | " | " | " | |
| 309 | $OCH_3$ | " | " | N | " | " | |
| 310 | " | " | O | " | " | " | |
| 311 | " | " | " | " | t-Bu | " | |
| 312 | Cl | " | " | " | " | " | |
| 313 | $CH_3$ | " | " | " | " | " | |
| 314 | $NHCH_3$ | " | " | " | " | " | |
| 315 | $OCH_3$ | " | " | " | " | " | |
| 316 | Cl | " | " | " | " | " | |
| 317 | $CH_3$ | " | " | " | " | " | |
| 318 | $NHCH_3$ | " | " | " | " | " | |
| 319 | $OCH_3$ | " | " | N | " | " | |
| 320 | " | " | O | " | " | " | |
| 321 | " | " | " | CH | Cyclopentyl | " | |
| 322 | Cl | " | " | " | " | " | |
| 323 | $CH_3$ | " | " | " | " | " | |
| 324 | $NHCH_3$ | " | " | " | " | " | |
| 325 | $OCH_3$ | " | S | " | " | " | |
| 326 | Cl | " | " | " | " | " | |
| 327 | $CH_3$ | " | " | " | " | " | |
| 328 | $NHCH_3$ | " | " | " | " | " | |
| 329 | $OCH_3$ | " | " | N | " | " | |
| 330 | " | " | O | " | " | " | |

| Bsp-Nr. | $R^1$ | $R^2$ | X | Y | $R^3$ | $R^4$ | Schmp.(C°) bzw. $[n]_D^{20}$ |
|---|---|---|---|---|---|---|---|
| 331 | $OCH_3$ | $OCH_3$ | O | CH | i-$C_3H_7$ | $OCH(CH_2)COSCH_2Ph$ | |
| 332 | Cl | " | " | " | " | " | |
| 333 | $CH_3$ | " | " | " | " | " | |
| 334 | $NHCH_3$ | " | " | " | " | " | |
| 335 | $OCH_3$ | " | S | " | " | " | |
| 336 | Cl | " | " | " | " | " | |
| 337 | $CH_3$ | " | " | " | " | " | |
| 338 | $NHCH_3$ | " | " | " | " | " | |
| 339 | $OCH_3$ | " | " | N | " | " | |
| 340 | " | " | O | " | " | " | |
| 341 | " | " | " | " | t-Bu | " | |
| 342 | Cl | " | " | " | " | " | |
| 343 | $CH_3$ | " | " | " | " | " | |
| 344 | $NHCH_3$ | " | " | " | " | " | |
| 345 | $OCH_3$ | " | " | " | " | " | |
| 346 | Cl | " | " | " | " | " | |
| 347 | $CH_3$ | " | " | " | " | " | |
| 348 | $NHCH_3$ | " | " | " | " | " | |
| 349 | $OCH_3$ | " | " | N | " | " | |
| 350 | " | " | O | " | " | " | |
| 351 | " | " | " | CH | Cyclopentyl | " | |
| 352 | Cl | " | " | " | " | " | |
| 353 | $CH_3$ | " | " | " | " | " | |
| 354 | $NHCH_3$ | " | " | " | " | " | |
| 355 | $OCH_3$ | " | S | " | " | " | |
| 356 | Cl | " | " | " | " | " | |
| 357 | $CH_3$ | " | " | " | " | " | |
| 358 | $NHCH_3$ | " | " | " | " | " | |
| 359 | $OCH_3$ | " | " | N | " | " | |
| 360 | " | " | O | " | " | " | |

| Bsp-Nr. | $R^1$ | $R^2$ | X | Y | $R^3$ | $R^4$ | Schmp.(C°) bzw.$[n]_D^{20}$ |
|---|---|---|---|---|---|---|---|
| 361 | $OCH_3$ | $OCH_3$ | O | CH | i-$C_3H_7$ | 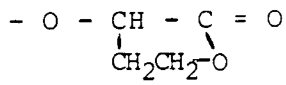 | 131 |
| 362 | Cl | " | " | " | " | " | |
| 363 | $CH_3$ | " | " | " | " | " | |
| 364 | $NHCH_3$ | " | " | " | " | " | |
| 365 | $OCH_3$ | " | S | " | " | " | |
| 366 | Cl | " | " | " | " | " | |
| 367 | $CH_3$ | " | " | " | " | " | |
| 368 | $NHCH_3$ | " | " | " | " | " | |
| 369 | $OCH_3$ | " | " | N | " | " | |
| 370 | " | " | O | " | " | " | |
| 371 | " | " | " | " | t-Bu | " | |
| 372 | Cl | " | " | " | " | " | |
| 373 | $CH_3$ | " | " | " | " | " | |
| 374 | $NHCH_3$ | " | " | " | " | " | |
| 375 | $OCH_3$ | " | " | " | " | " | |
| 376 | Cl | " | " | " | " | " | |
| 377 | $CH_3$ | " | " | " | " | " | |
| 378 | $NHCH_3$ | " | " | " | " | " | |
| 379 | $OCH_3$ | " | " | N | " | " | |
| 380 | " | " | O | " | " | " | |
| 381 | " | " | " | CH | Cyclopentyl | " | |
| 382 | Cl | " | " | " | " | " | |
| 383 | $CH_3$ | " | " | " | " | " | |
| 384 | $NHCH_3$ | " | " | " | " | " | |
| 385 | $OCH_3$ | " | S | " | " | " | |
| 386 | Cl | " | " | " | " | " | |
| 387 | $CH_3$ | " | " | " | " | " | |
| 388 | $NHCH_3$ | " | " | " | " | " | |
| 389 | $OCH_3$ | " | " | N | " | " | |
| 390 | " | " | O | " | " | " | |

| Bsp-Nr. | $R^1$ | $R^2$ | X | Y | $R^3$ | $R^4$ | Schmp.(C°) bzw. $[n]_D^{20}$ |
|---|---|---|---|---|---|---|---|
| 391 | $OCH_3$ | $OCH_3$ | O | CH | i-$C_3H_7$ | $OCH_2Si(CH_3)_3$ | Öl |
| 392 | Cl | " | " | " | " | " | |
| 393 | $CH_3$ | " | " | " | " | " | |
| 394 | $NHCH_3$ | " | " | " | " | " | |
| 395 | $OCH_3$ | " | S | " | " | " | |
| 396 | Cl | " | " | " | " | " | |
| 397 | $CH_3$ | " | " | " | " | " | |
| 398 | $NHCH_3$ | " | " | " | " | " | |
| 399 | $OCH_3$ | " | " | N | " | " | |
| 400 | " | " | O | " | " | " | |
| 401 | " | " | " | " | t-Bu | " | |
| 402 | Cl | " | " | " | " | " | |
| 403 | $CH_3$ | " | " | " | " | " | |
| 404 | $NHCH_3$ | " | " | " | " | " | |
| 405 | $OCH_3$ | " | " | " | " | " | |
| 406 | Cl | " | " | " | " | " | |
| 407 | $CH_3$ | " | " | " | " | " | |
| 408 | $NHCH_3$ | " | " | " | " | " | |
| 409 | $OCH_3$ | " | " | N | " | " | |
| 410 | " | " | O | " | " | " | |
| 411 | " | " | " | CH | Cyclopentyl | " | |
| 412 | Cl | " | " | " | " | " | |
| 413 | $CH_3$ | " | " | " | " | " | |
| 414 | $NHCH_3$ | " | " | " | " | " | |
| 415 | $OCH_3$ | " | S | " | " | " | |
| 416 | Cl | " | " | " | " | " | |
| 417 | $CH_3$ | " | " | " | " | " | |
| 418 | $NHCH_3$ | " | " | " | " | " | |
| 419 | $OCH_3$ | " | " | N | " | " | |
| 420 | " | " | O | " | " | " | |

| Bsp-Nr. | $R^1$ | $R^2$ | X | Y | $R^3$ | $R^4$ | Schmp.(C°) bzw.$[n]_D^{20}$ |
|---|---|---|---|---|---|---|---|
| 421 | $OCH_3$ | $OCH_3$ | O | CH | i-$C_3H_7$ | Pyrid-2-ylmethoxy | |
| 422 | Cl | " | " | " | " | " | |
| 423 | $CH_3$ | " | " | " | " | " | |
| 424 | $NHCH_3$ | " | " | " | " | " | |
| 425 | $OCH_3$ | " | S | " | " | " | |
| 426 | Cl | " | " | " | " | " | |
| 427 | $CH_3$ | " | " | " | " | " | |
| 428 | $NHCH_3$ | " | " | " | " | " | |
| 429 | $OCH_3$ | " | " | N | " | " | |
| 430 | " | " | O | " | " | " | |
| 431 | " | " | " | " | t-Bu | " | |
| 432 | Cl | " | " | " | " | " | |
| 433 | $CH_3$ | " | " | " | " | " | |
| 434 | $NHCH_3$ | " | " | " | " | " | |
| 435 | $OCH_3$ | " | " | " | " | " | |
| 436 | Cl | " | " | " | " | " | |
| 437 | $CH_3$ | " | " | " | " | " | |
| 438 | $NHCH_3$ | " | " | " | " | " | |
| 439 | $OCH_3$ | " | " | N | " | " | |
| 440 | " | " | O | " | " | " | |
| 441 | " | " | " | CH | Cyclopentyl | " | 88-90 |
| 442 | Cl | " | " | " | " | " | |
| 443 | $CH_3$ | " | " | " | " | " | |
| 444 | $NHCH_3$ | " | " | " | " | " | |
| 445 | $OCH_3$ | " | S | " | " | " | |
| 446 | Cl | " | " | " | " | " | |
| 447 | $CH_3$ | " | " | " | " | " | |
| 448 | $NHCH_3$ | " | " | " | " | " | |
| 449 | $OCH_3$ | " | " | N | " | " | |
| 450 | " | " | O | " | " | " | |

| Bsp-Nr. | $R^1$ | $R^2$ | X | Y | $R^3$ | $R^4$ | Schmp.(C°) bzw.$[n]_D^{20}$ |
|---|---|---|---|---|---|---|---|
| 451 | $OCH_3$ | $OCH_3$ | O | CH | $i\text{-}C_3H_7$ | Pyrid-3-ylmethoxy | |
| 452 | Cl | " | " | " | " | " | |
| 453 | $CH_3$ | " | " | " | " | " | |
| 454 | $NHCH_3$ | " | " | " | " | " | |
| 455 | $OCH_3$ | " | S | " | " | " | |
| 456 | Cl | " | " | " | " | " | |
| 457 | $CH_3$ | " | " | " | " | " | |
| 458 | $NHCH_3$ | " | " | " | " | " | |
| 459 | $OCH_3$ | " | " | N | " | " | |
| 460 | " | " | O | " | " | " | |
| 461 | " | " | " | " | t-Bu | " | |
| 462 | Cl | " | " | " | " | " | |
| 463 | $CH_3$ | " | " | " | " | " | |
| 464 | $NHCH_3$ | " | " | " | " | " | |
| 465 | $OCH_3$ | " | " | " | " | " | |
| 466 | Cl | " | " | " | " | " | |
| 467 | $CH_3$ | " | " | " | " | " | |
| 468 | $NHCH_3$ | " | " | " | " | " | |
| 469 | $OCH_3$ | " | " | N | " | " | |
| 470 | " | " | O | " | " | " | |
| 471 | " | " | " | CH | Cyclopentyl | " | |
| 472 | Cl | " | " | " | " | " | |
| 473 | $CH_3$ | " | " | " | " | " | |
| 474 | $NHCH_3$ | " | " | " | " | " | |
| 475 | $OCH_3$ | " | S | " | " | " | |
| 476 | Cl | " | " | " | " | " | |
| 477 | $CH_3$ | " | " | " | " | " | |
| 478 | $NHCH_3$ | " | " | " | " | " | |
| 479 | $OCH_3$ | " | " | N | " | " | |
| 480 | " | " | O | " | " | " | |

| Bsp-Nr. | $R^1$ | $R^2$ | X | Y | $R^3$ | $R^4$ | Schmp.(C°) bzw. $[n]_D^{20}$ |
|---|---|---|---|---|---|---|---|
| 481 | $OCH_3$ | $OCH_3$ | O | CH | $i\text{-}C_3H_7$ | $O\text{-}CH_2\text{-}P(=O)(CH_3)_2$ | |
| 482 | Cl | " | " | " | " | " | |
| 483 | $CH_3$ | " | " | " | " | " | |
| 484 | $NHCH_3$ | " | " | " | " | " | |
| 485 | $OCH_3$ | " | S | " | " | " | |
| 486 | Cl | " | " | " | " | " | |
| 487 | $CH_3$ | " | " | " | " | " | |
| 488 | $NHCH_3$ | " | " | " | " | " | |
| 489 | $OCH_3$ | " | " | N | " | " | |
| 490 | " | " | O | " | " | " | |
| 491 | " | " | " | " | t-Bu | " | |
| 492 | Cl | " | " | " | " | " | |
| 493 | $CH_3$ | " | " | " | " | " | |
| 494 | $NHCH_3$ | " | " | " | " | " | |
| 495 | $OCH_3$ | " | " | " | " | " | |
| 496 | Cl | " | " | " | " | " | |
| 497 | $CH_3$ | " | " | " | " | " | |
| 498 | $NHCH_3$ | " | " | " | " | " | |
| 499 | $OCH_3$ | " | " | N | " | " | |
| 500 | " | " | O | " | " | " | |
| 501 | " | " | " | CH | Cyclopentyl | " | Öl |
| 502 | Cl | " | " | " | " | " | |
| 503 | $CH_3$ | " | " | " | " | " | |
| 504 | $NHCH_3$ | " | " | " | " | " | |
| 505 | $OCH_3$ | " | S | " | " | " | |
| 506 | Cl | " | " | " | " | " | |
| 507 | $CH_3$ | " | " | " | " | " | |
| 508 | $NHCH_3$ | " | " | " | " | " | |
| 509 | $OCH_3$ | " | " | N | " | " | |
| 510 | " | " | O | " | " | " | |

| Bsp-Nr. | $R^1$ | $R^2$ | X | Y | $R^3$ | $R^4$ | Schmp.(C°) bzw.$[n]_D^{20}$ |
|---|---|---|---|---|---|---|---|
| 511 | $OCH_3$ | $OCH_3$ | O | CH | i-$C_3H_7$ | O-Si$(CH_3)_2$(t-Bu) | Öl |
| 512 | Cl | " | " | " | " | " | |
| 513 | $CH_3$ | " | " | " | " | " | |
| 514 | $NHCH_3$ | " | " | " | " | " | |
| 515 | $OCH_3$ | " | S | " | " | " | |
| 516 | Cl | " | " | " | " | " | |
| 517 | $CH_3$ | " | " | " | " | " | |
| 518 | $NHCH_3$ | " | " | " | " | " | |
| 519 | $OCH_3$ | " | " | N | " | " | |
| 520 | " | " | O | " | " | " | |
| 521 | " | " | " | " | t-Bu | " | |
| 522 | Cl | " | " | " | " | " | |
| 523 | $CH_3$ | " | " | " | " | " | |
| 524 | $NHCH_3$ | " | " | " | " | " | |
| 525 | $OCH_3$ | " | " | " | " | " | |
| 526 | Cl | " | " | " | " | " | |
| 527 | $CH_3$ | " | " | " | " | " | |
| 528 | $NHCH_3$ | " | " | " | " | " | |
| 529 | $OCH_3$ | " | " | N | " | " | |
| 530 | " | " | O | " | " | " | |
| 531 | " | " | " | CH | Cyclopentyl | " | |
| 532 | Cl | " | " | " | " | " | |
| 533 | $CH_3$ | " | " | " | " | " | |
| 534 | $NHCH_3$ | " | " | " | " | " | |
| 535 | $OCH_3$ | " | S | " | " | " | |
| 536 | Cl | " | " | " | " | " | |
| 537 | $CH_3$ | " | " | " | " | " | |
| 538 | $NHCH_3$ | " | " | " | " | " | |
| 539 | $OCH_3$ | " | " | N | " | " | |
| 540 | " | " | O | " | " | " | |

| Bsp-Nr. | $R^1$ | $R^2$ | X | Y | $R^3$ | $R^4$ | Schmp.(C°) bzw.$[n]_D^{20}$ |
|---|---|---|---|---|---|---|---|
| 541 | $OCH_3$ | $OCH_3$ | O | CH | i-$C_3H_7$ | O-Si(i-Pr)$_3$ | 1,4800 |
| 542 | Cl | " | " | " | " | " | |
| 543 | $CH_3$ | " | " | " | " | " | |
| 544 | $NHCH_3$ | " | " | " | " | " | |
| 545 | $OCH_3$ | " | S | " | " | " | |
| 546 | Cl | " | " | " | " | " | |
| 547 | $CH_3$ | " | " | " | " | " | |
| 548 | $NHCH_3$ | " | " | " | " | " | |
| 549 | $OCH_3$ | " | " | N | " | " | |
| 550 | " | " | O | " | " | " | |
| 551 | " | " | " | " | t-Bu | " | |
| 552 | Cl | " | " | " | " | " | |
| 553 | $CH_3$ | " | " | " | " | " | |
| 554 | $NHCH_3$ | " | " | " | " | " | |
| 555 | $OCH_3$ | " | " | " | " | " | |
| 556 | Cl | " | " | " | " | " | |
| 557 | $CH_3$ | " | " | " | " | " | |
| 558 | $NHCH_3$ | " | " | " | " | " | |
| 559 | $OCH_3$ | " | " | N | " | " | |
| 560 | " | " | O | " | " | " | |
| 561 | " | " | " | CH | Cyclopentyl | " | |
| 562 | Cl | " | " | " | " | " | |
| 563 | $CH_3$ | " | " | " | " | " | |
| 564 | $NHCH_3$ | " | " | " | " | " | |
| 565 | $OCH_3$ | " | S | " | " | " | |
| 566 | Cl | " | " | " | " | " | |
| 567 | $CH_3$ | " | " | " | " | " | |
| 568 | $NHCH_3$ | " | " | " | " | " | |
| 569 | $OCH_3$ | " | " | N | " | " | |
| 570 | " | " | O | " | " | " | |

| Bsp-Nr. | $R^1$ | $R^2$ | X | Y | $R^3$ | $R^4$ | Schmp.(C°) bzw.$[n]_D^{20}$ |
|---|---|---|---|---|---|---|---|
| 571 | $OCH_3$ | $OCH_3$ | O | CH | i-$C_3H_7$ | $-OCH_2C(OCH_3)=CHCOOCH_3$ | 77-78 |
| 572 | Cl | " | " | " | " | " | |
| 573 | $CH_3$ | " | " | " | " | " | |
| 574 | $NHCH_3$ | " | " | " | " | " | |
| 575 | $OCH_3$ | " | S | " | " | " | |
| 576 | Cl | " | " | " | " | " | |
| 577 | $CH_3$ | " | " | " | " | " | |
| 578 | $NHCH_3$ | " | " | " | " | " | |
| 579 | $OCH_3$ | " | " | N | " | " | |
| 580 | " | " | O | " | " | " | |
| 581 | " | " | " | " | t-Bu | " | |
| 582 | Cl | " | " | " | " | " | |
| 583 | $CH_3$ | " | " | " | " | " | |
| 584 | $NHCH_3$ | " | " | " | " | " | |
| 585 | $OCH_3$ | " | " | " | " | " | |
| 586 | Cl | " | " | " | " | " | |
| 587 | $CH_3$ | " | " | " | " | " | |
| 588 | $NHCH_3$ | " | " | " | " | " | |
| 589 | $OCH_3$ | " | " | N | " | " | |
| 590 | " | " | O | " | " | " | |
| 591 | " | " | " | CH | Cyclopentyl | " | 72-74 |
| 592 | Cl | " | " | " | " | " | |
| 593 | $CH_3$ | " | " | " | " | " | |
| 594 | $NHCH_3$ | " | " | " | " | " | |
| 595 | $OCH_3$ | " | S | " | " | " | |
| 596 | Cl | " | " | " | " | " | |
| 597 | $CH_3$ | " | " | " | " | " | |
| 598 | $NHCH_3$ | " | " | " | " | " | |
| 599 | $OCH_3$ | " | " | N | " | " | |
| 600 | " | " | O | " | " | " | |

| Bsp-Nr. | $R^1$ | $R^2$ | X | Y | $R^3$ | $R^4$ | Schmp.(C°) bzw.$[n]_D^{20}$ |
|---|---|---|---|---|---|---|---|
| 601 | $OCH_3$ | $OCH_3$ | O | CH | i-$C_3H_7$ | O-$CH_2COCH_2COOCH_3$ | Öl |
| 602 | Cl | " | " | " | " | " | |
| 603 | $CH_3$ | " | " | " | " | " | |
| 604 | $NHCH_3$ | " | " | " | " | " | |
| 605 | $OCH_3$ | " | S | " | " | " | |
| 606 | Cl | " | " | " | " | " | |
| 607 | $CH_3$ | " | " | " | " | " | |
| 608 | $NHCH_3$ | " | " | " | " | " | |
| 609 | $OCH_3$ | " | " | N | " | " | |
| 610 | " | " | O | " | " | " | |
| 611 | " | " | " | " | t-Bu | " | |
| 612 | Cl | " | " | " | " | " | |
| 613 | $CH_3$ | " | " | " | " | " | |
| 614 | $NHCH_3$ | " | " | " | " | " | |
| 615 | $OCH_3$ | " | " | " | " | " | |
| 616 | Cl | " | " | " | " | " | |
| 617 | $CH_3$ | " | " | " | " | " | |
| 618 | $NHCH_3$ | " | " | " | " | " | |
| 619 | $OCH_3$ | " | " | N | " | " | |
| 620 | " | " | O | " | " | " | |
| 621 | " | " | " | CH | Cyclopentyl | " | 72-74 |
| 622 | Cl | " | " | " | " | " | |
| 623 | $CH_3$ | " | " | " | " | " | |
| 624 | $NHCH_3$ | " | " | " | " | " | |
| 625 | $OCH_3$ | " | S | " | " | " | |
| 626 | Cl | " | " | " | " | " | |
| 627 | $CH_3$ | " | " | " | " | " | |
| 628 | $NHCH_3$ | " | " | " | " | " | |
| 629 | $OCH_3$ | " | " | N | " | " | |
| 630 | " | " | O | " | " | " | |

| Bsp-Nr. | $R^1$ | $R^2$ | X | Y | $R^3$ | $R^4$ | Schmp.(C°) bzw.$[n]_D^{20}$ |
|---|---|---|---|---|---|---|---|
| 631 | $OCH_3$ | $OCH_3$ | O | CH | i-$C_3H_7$ | $OCH_2SO_2CH_3$ | 108-110 |
| 632 | Cl | " | " | " | " | " | |
| 633 | $CH_3$ | " | " | " | " | " | |
| 634 | $NHCH_3$ | " | " | " | " | " | |
| 635 | $OCH_3$ | " | S | " | " | " | |
| 636 | Cl | " | " | " | " | " | |
| 637 | $CH_3$ | " | " | " | " | " | |
| 638 | $NHCH_3$ | " | " | " | " | " | |
| 639 | $OCH_3$ | " | " | N | " | " | |
| 640 | " | " | O | " | " | " | |
| 641 | " | " | " | " | t-Bu | " | |
| 642 | Cl | " | " | " | " | " | |
| 643 | $CH_3$ | " | " | " | " | " | |
| 644 | $NHCH_3$ | " | " | " | " | " | |
| 645 | $OCH_3$ | " | " | " | " | " | |
| 646 | Cl | " | " | " | " | " | |
| 647 | $CH_3$ | " | " | " | " | " | |
| 648 | $NHCH_3$ | " | " | " | " | " | |
| 649 | $OCH_3$ | " | " | N | " | " | |
| 650 | " | " | O | " | " | " | |
| 651 | " | " | " | CH | Cyclopentyl | " | |
| 652 | Cl | " | " | " | " | " | |
| 653 | $CH_3$ | " | " | " | " | " | |
| 654 | $NHCH_3$ | " | " | " | " | " | |
| 655 | $OCH_3$ | " | S | " | " | " | |
| 656 | Cl | " | " | " | " | " | |
| 657 | $CH_3$ | " | " | " | " | " | |
| 658 | $NHCH_3$ | " | " | " | " | " | |
| 659 | $OCH_3$ | " | " | N | " | " | |
| 660 | " | " | O | " | " | " | |

| Bsp-Nr. | $R^1$ | $R^2$ | X | Y | $R^3$ | $R^4$ | Schmp.(C°) bzw. $[n]_D^{20}$ |
|---|---|---|---|---|---|---|---|
| 661 | $OCH_3$ | $OCH_3$ | O | CH | i-$C_3H_7$ | $OCH_2S(O)CH_2CH_3$ | |
| 662 | Cl | " | " | " | " | " | |
| 663 | $CH_3$ | " | " | " | " | " | |
| 664 | $NHCH_3$ | " | " | " | " | " | |
| 665 | $OCH_3$ | " | S | " | " | " | |
| 666 | Cl | " | " | " | " | " | |
| 667 | $CH_3$ | " | " | " | " | " | |
| 668 | $NHCH_3$ | " | " | " | " | " | |
| 669 | $OCH_3$ | " | " | N | " | " | |
| 670 | " | " | O | " | " | " | |
| 671 | " | " | " | " | t-Bu | " | |
| 672 | Cl | " | " | " | " | " | |
| 673 | $CH_3$ | " | " | " | " | " | |
| 674 | $NHCH_3$ | " | " | " | " | " | |
| 675 | $OCH_3$ | " | " | " | " | " | |
| 676 | Cl | " | " | " | " | " | |
| 677 | $CH_3$ | " | " | " | " | " | |
| 678 | $NHCH_3$ | " | " | " | " | " | |
| 679 | $OCH_3$ | " | " | N | " | " | |
| 680 | " | " | O | " | " | " | |
| 681 | " | " | " | CH | Cyclopentyl | " | |
| 682 | Cl | " | " | " | " | " | |
| 683 | $CH_3$ | " | " | " | " | " | |
| 684 | $NHCH_3$ | " | " | " | " | " | |
| 685 | $OCH_3$ | " | S | " | " | " | |
| 686 | Cl | " | " | " | " | " | |
| 687 | $CH_3$ | " | " | " | " | " | |
| 688 | $NHCH_3$ | " | " | " | " | " | |
| 689 | $OCH_3$ | " | " | N | " | " | |
| 690 | " | " | O | " | " | " | |

| Bsp-Nr. | $R^1$ | $R^2$ | X | Y | $R^3$ | $R^4$ | Schmp.(C°) bzw.$[n]_D^{20}$ |
|---|---|---|---|---|---|---|---|
| 691 | $OCH_3$ | $OCH_3$ | O | CH | $i-C_3H_7$ | $OCH_2SOCH_3$ | 76-78 |
| 692 | Cl | " | " | " | " | " | |
| 693 | $CH_3$ | " | " | " | " | " | |
| 694 | $NHCH_3$ | " | " | " | " | " | |
| 695 | $OCH_3$ | " | S | " | " | " | |
| 696 | Cl | " | " | " | " | " | |
| 697 | $CH_3$ | " | " | " | " | " | |
| 698 | $NHCH_3$ | " | " | " | " | " | |
| 699 | $OCH_3$ | " | " | N | " | " | |
| 700 | " | " | O | " | " | " | |
| 701 | " | " | " | " | t-Bu | " | |
| 702 | Cl | " | " | " | " | " | |
| 703 | $CH_3$ | " | " | " | " | " | |
| 704 | $NHCH_3$ | " | " | " | " | " | |
| 705 | $OCH_3$ | " | " | " | " | " | |
| 706 | Cl | " | " | " | " | " | |
| 707 | $CH_3$ | " | " | " | " | " | |
| 708 | $NHCH_3$ | " | " | " | " | " | |
| 709 | $OCH_3$ | " | " | N | " | " | |
| 710 | " | " | O | " | " | " | |
| 711 | " | " | " | CH | Cyclopentyl | " | |
| 712 | Cl | " | " | " | " | " | |
| 713 | $CH_3$ | " | " | " | " | " | |
| 714 | $NHCH_3$ | " | " | " | " | " | |
| 715 | $OCH_3$ | " | S | " | " | " | |
| 716 | Cl | " | " | " | " | " | |
| 717 | $CH_3$ | " | " | " | " | " | |
| 718 | $NHCH_3$ | " | " | " | " | " | |
| 719 | $OCH_3$ | " | " | N | " | " | |
| 720 | " | " | O | " | " | " | |

| Bsp-Nr. | $R^1$ | $R^2$ | X | Y | $R^3$ | $R^4$ | Schmp.(C°) bzw.$[n]_D^{20}$ |
|---|---|---|---|---|---|---|---|
| 721 | $OCH_3$ | $OCH_3$ | O | CH | i-$C_3H_7$ | $OCH_2SO_2C_2H_5$ | |
| 722 | Cl | " | " | " | " | " | |
| 723 | $CH_3$ | " | " | " | " | " | |
| 724 | $NHCH_3$ | " | " | " | " | " | |
| 725 | $OCH_3$ | " | S | " | " | " | |
| 726 | Cl | " | " | " | " | " | |
| 727 | $CH_3$ | " | " | " | " | " | |
| 728 | $NHCH_3$ | " | " | " | " | " | |
| 729 | $OCH_3$ | " | " | N | " | " | |
| 730 | " | " | O | " | " | " | |
| 731 | " | " | " | " | t-Bu | " | |
| 732 | Cl | " | " | " | " | " | |
| 733 | $CH_3$ | " | " | " | " | " | |
| 734 | $NHCH_3$ | " | " | " | " | " | |
| 735 | $OCH_3$ | " | " | " | " | " | |
| 736 | Cl | " | " | " | " | " | |
| 737 | $CH_3$ | " | " | " | " | " | |
| 738 | $NHCH_3$ | " | " | " | " | " | |
| 739 | $OCH_3$ | " | " | N | " | " | |
| 740 | " | " | O | " | " | " | |
| 741 | " | " | " | CH | Cyclopentyl | " | |
| 742 | Cl | " | " | " | " | " | |
| 743 | $CH_3$ | " | " | " | " | " | |
| 744 | $NHCH_3$ | " | " | " | " | " | |
| 745 | $OCH_3$ | " | S | " | " | " | |
| 746 | Cl | " | " | " | " | " | |
| 747 | $CH_3$ | " | " | " | " | " | |
| 748 | $NHCH_3$ | " | " | " | " | " | |
| 749 | $OCH_3$ | " | " | N | " | " | |
| 750 | " | " | O | " | " | " | |

| Bsp-Nr. | $R^1$ | $R^2$ | X | Y | $R^3$ | $R^4$ | Schmp.(C°) bzw.$[n]_D^{20}$ |
|---|---|---|---|---|---|---|---|
| 751 | $OCH_3$ | $OCH_3$ | O | CH | i-$C_3H_7$ | $OCH_2CH_2SO_2C_2H_5$ | |
| 752 | Cl | " | " | " | " | " | |
| 753 | $CH_3$ | " | " | " | " | " | |
| 754 | $NHCH_3$ | " | " | " | " | " | |
| 755 | $OCH_3$ | " | S | " | " | " | |
| 756 | Cl | " | " | " | " | " | |
| 757 | $CH_3$ | " | " | " | " | " | |
| 758 | $NHCH_3$ | " | " | " | " | " | |
| 759 | $OCH_3$ | " | " | N | " | " | |
| 760 | " | " | O | " | " | " | |
| 761 | " | " | " | " | t-Bu | " | |
| 762 | Cl | " | " | " | " | " | |
| 763 | $CH_3$ | " | " | " | " | " | |
| 764 | $NHCH_3$ | " | " | " | " | " | |
| 765 | $OCH_3$ | " | " | " | " | " | |
| 766 | Cl | " | " | " | " | " | |
| 767 | $CH_3$ | " | " | " | " | " | |
| 768 | $NHCH_3$ | " | " | " | " | " | |
| 769 | $OCH_3$ | " | " | N | " | " | |
| 770 | " | " | O | " | " | " | |
| 771 | " | " | " | CH | Cycopentyl | " | |
| 772 | Cl | " | " | " | " | " | |
| 773 | $CH_3$ | " | " | " | " | " | |
| 774 | $NHCH_3$ | " | " | " | " | " | |
| 775 | $OCH_3$ | " | S | " | " | " | |
| 776 | Cl | " | " | " | " | " | |
| 777 | $CH_3$ | " | " | " | " | " | |
| 778 | $NHCH_3$ | " | " | " | " | " | |
| 779 | $OCH_3$ | " | " | N | " | " | |
| 780 | " | " | O | " | " | " | |

| Bsp-Nr. | $R^1$ | $R^2$ | X | Y | $R^3$ | $R^4$ | Schmp.(C°) bzw.$[n]_D^{20}$ |
|---|---|---|---|---|---|---|---|
| 781 | $OCH_3$ | $OCH_3$ | O | CH | i-$C_3H_7$ | $OC_3H_6OCH_2CH=CH_2$ | |
| 782 | Cl | " | " | " | " | " | |
| 783 | $CH_3$ | " | " | " | " | " | |
| 784 | $NHCH_3$ | " | " | " | " | " | |
| 785 | $OCH_3$ | " | S | " | " | " | |
| 786 | Cl | " | " | " | " | " | |
| 787 | $CH_3$ | " | " | " | " | " | |
| 788 | $NHCH_3$ | " | " | " | " | " | |
| 789 | $OCH_3$ | " | " | N | " | " | |
| 790 | " | " | O | " | " | " | |
| 791 | " | " | " | " | t-Bu | " | |
| 792 | Cl | " | " | " | " | " | |
| 793 | $CH_3$ | " | " | " | " | " | |
| 794 | $NHCH_3$ | " | " | " | " | " | |
| 795 | $OCH_3$ | " | " | " | " | " | |
| 796 | Cl | " | " | " | " | " | |
| 797 | $CH_3$ | " | " | " | " | " | |
| 798 | $NHCH_3$ | " | " | " | " | " | |
| 799 | $OCH_3$ | " | " | N | " | " | |
| 801 | " | " | O | " | " | " | |
| 801 | " | " | " | CH | Cyclopentyl | " | |
| 802 | Cl | " | " | " | " | " | |
| 803 | $CH_3$ | " | " | " | " | " | |
| 804 | $NHCH_3$ | " | " | " | " | " | |
| 805 | $OCH_3$ | " | S | " | " | " | |
| 806 | Cl | " | " | " | " | " | |
| 807 | $CH_3$ | " | " | " | " | " | |
| 808 | $NHCH_3$ | " | " | " | " | " | |
| 809 | $OCH_3$ | " | " | N | " | " | |
| 810 | " | " | O | " | " | " | |

| Bsp-Nr. | $R^1$ | $R^2$ | X | Y | $R^3$ | $R^4$ | Schmp.(C°) bzw.$[n]_D^{20}$ |
|---|---|---|---|---|---|---|---|
| 811 | $OCH_3$ | $OCH_3$ | O | CH | i-$C_3H_7$ | $OCH_2CH_2OCH_2Ph$ | |
| 812 | Cl | " | " | " | " | " | |
| 813 | $CH_3$ | " | " | " | " | " | |
| 814 | $NHCH_3$ | " | " | " | " | " | |
| 815 | $OCH_3$ | " | S | " | " | " | |
| 816 | Cl | " | " | " | " | " | |
| 817 | $CH_3$ | " | " | " | " | " | |
| 818 | $NHCH_3$ | " | " | " | " | " | |
| 819 | $OCH_3$ | " | " | N | " | " | |
| 820 | " | " | O | " | " | " | |
| 821 | " | " | " | " | t-Bu | " | |
| 822 | Cl | " | " | " | " | " | |
| 823 | $CH_3$ | " | " | " | " | " | |
| 824 | $NHCH_3$ | " | " | " | " | " | |
| 825 | $OCH_3$ | " | " | " | " | " | |
| 826 | Cl | " | " | " | " | " | |
| 827 | $CH_3$ | " | " | " | " | " | |
| 828 | $NHCH_3$ | " | " | " | " | " | |
| 829 | $OCH_3$ | " | " | N | " | " | |
| 830 | " | " | O | " | " | " | |
| 831 | " | " | " | CH | Cyclopentyl | " | |
| 832 | Cl | " | " | " | " | " | |
| 833 | $CH_3$ | " | " | " | " | " | |
| 834 | $NHCH_3$ | " | " | " | " | " | |
| 835 | $OCH_3$ | " | S | " | " | " | |
| 836 | Cl | " | " | " | " | " | |
| 837 | $CH_3$ | " | " | " | " | " | |
| 838 | $NHCH_3$ | " | " | " | " | " | |
| 839 | $OCH_3$ | " | " | N | " | " | |
| 840 | " | " | O | " | " | " | |

| Bsp-Nr. | $R^1$ | $R^2$ | X | Y | $R^3$ | $R^4$ | Schmp.(C°) bzw.$[n]_D^{20}$ |
|---|---|---|---|---|---|---|---|
| 841 | $OCH_3$ | $OCH_3$ | O | CH | i-$C_3H_7$ | $OCH_2CH_2$-O-$CH_2C\equiv CH$ | |
| 842 | Cl | " | " | " | " | " | |
| 843 | $CH_3$ | " | " | " | " | " | |
| 844 | $NHCH_3$ | " | " | " | " | " | |
| 845 | $OCH_3$ | " | S | " | " | " | |
| 846 | Cl | " | " | " | " | " | |
| 847 | $CH_3$ | " | " | " | " | " | |
| 848 | $NHCH_3$ | " | " | " | " | " | |
| 849 | $OCH_3$ | " | " | N | " | " | |
| 850 | " | " | O | " | " | " | |
| 851 | " | " | " | " | t-Bu | " | |
| 852 | Cl | " | " | " | " | " | |
| 853 | $CH_3$ | " | " | " | " | " | |
| 854 | $NHCH_3$ | " | " | " | " | " | |
| 855 | $OCH_3$ | " | " | " | " | " | |
| 856 | Cl | " | " | " | " | " | |
| 857 | $CH_3$ | " | " | " | " | " | |
| 858 | $NHCH_3$ | " | " | " | " | " | |
| 859 | $OCH_3$ | " | " | N | " | " | |
| 860 | " | " | O | " | " | " | |
| 861 | " | " | " | CH | Cyclopentyl | " | |
| 862 | Cl | " | " | " | " | " | |
| 863 | $CH_3$ | " | " | " | " | " | |
| 864 | $NHCH_3$ | " | " | " | " | " | |
| 865 | $OCH_3$ | " | S | " | " | " | |
| 866 | Cl | " | " | " | " | " | |
| 867 | $CH_3$ | " | " | " | " | " | |
| 868 | $NHCH_3$ | " | " | " | " | " | |
| 869 | $OCH_3$ | " | " | N | " | " | |
| 870 | " | " | O | " | " | " | |

| Bsp-Nr. | $R^1$ | $R^2$ | X | Y | $R^3$ | $R^4$ | Schmp.(C°) bzw.$[n]_D^{20}$ |
|---|---|---|---|---|---|---|---|
| 871 | $OCH_3$ | $OCH_3$ | O | CH | i-$C_3H_7$ | -$OCH_2CH(CH_3)OCH_2CH=CH_2$ | Öl |
| 872 | Cl | " | " | " | " | " | |
| 873 | $CH_3$ | " | " | " | " | " | |
| 874 | $NHCH_3$ | " | " | " | " | " | |
| 875 | $OCH_3$ | " | S | " | " | " | |
| 876 | Cl | " | " | " | " | " | |
| 877 | $CH_3$ | " | " | " | " | " | |
| 878 | $NHCH_3$ | " | " | " | " | " | |
| 879 | $OCH_3$ | " | " | N | " | " | |
| 880 | " | " | O | " | " | " | |
| 881 | " | " | " | " | t-Bu | " | |
| 882 | Cl | " | " | " | " | " | |
| 883 | $CH_3$ | " | " | " | " | " | |
| 884 | $NHCH_3$ | " | " | " | " | " | |
| 885 | $OCH_3$ | " | " | " | " | " | |
| 886 | Cl | " | " | " | " | " | |
| 887 | $CH_3$ | " | " | " | " | " | |
| 888 | $NHCH_3$ | " | " | " | " | " | |
| 889 | $OCH_3$ | " | " | N | " | " | |
| 890 | " | " | O | " | " | " | |
| 891 | " | " | " | CH | Cyclopentyl | " | Öl |
| 892 | Cl | " | " | " | " | " | |
| 893 | $CH_3$ | " | " | " | " | " | |
| 894 | $NHCH_3$ | " | " | " | " | " | |
| 895 | $OCH_3$ | " | S | " | " | " | |
| 896 | Cl | " | " | " | " | " | |
| 897 | $CH_3$ | " | " | " | " | " | |
| 898 | $NHCH_3$ | " | " | " | " | " | |
| 899 | $OCH_3$ | " | " | N | " | " | |
| 900 | " | " | O | " | " | " | |

| Bsp-Nr. | $R^1$ | $R^2$ | X | Y | $R^3$ | $R^4$ | Schmp.(C°) bzw.$[n]_D^{20}$ |
|---|---|---|---|---|---|---|---|
| 901 | $OCH_3$ | $OCH_3$ | O | CH | $i\text{-}C_3H_7$ | $OC_4H_8\text{-}O\text{-}CH_2\text{-}CH=CH_2$ | |
| 902 | Cl | " | " | " | " | " | |
| 903 | $CH_3$ | " | " | " | " | " | |
| 904 | $NHCH_3$ | " | " | " | " | " | |
| 905 | $OCH_3$ | " | S | " | " | " | |
| 906 | Cl | " | " | " | " | " | |
| 907 | $CH_3$ | " | " | " | " | " | |
| 908 | $NHCH_3$ | " | " | " | " | " | |
| 909 | $OCH_3$ | " | " | N | " | " | |
| 910 | " | " | O | " | " | " | |
| 911 | " | " | " | " | t-Bu | " | |
| 912 | Cl | " | " | " | " | " | |
| 913 | $CH_3$ | " | " | " | " | " | |
| 914 | $NHCH_3$ | " | " | " | " | " | |
| 915 | $OCH_3$ | " | " | " | " | " | |
| 916 | Cl | " | " | " | " | " | |
| 917 | $CH_3$ | " | " | " | " | " | |
| 918 | $NHCH_3$ | " | " | " | " | " | |
| 919 | $OCH_3$ | " | " | N | " | " | |
| 920 | " | " | O | " | " | " | |
| 921 | " | " | " | CH | Cyclopentyl | " | |
| 922 | Cl | " | " | " | " | " | |
| 923 | $CH_3$ | " | " | " | " | " | |
| 924 | $NHCH_3$ | " | " | " | " | " | |
| 925 | $OCH_3$ | " | S | " | " | " | |
| 926 | Cl | " | " | " | " | " | |
| 927 | $CH_3$ | " | " | " | " | " | |
| 928 | $NHCH_3$ | " | " | " | " | " | |
| 929 | $OCH_3$ | " | " | N | " | " | |
| 930 | " | " | O | " | " | " | |

| Bsp-Nr. | $R^1$ | $R^2$ | X | Y | $R^3$ | $R^4$ | Schmp.(C°) bzw. $[n]_D^{20}$ |
|---|---|---|---|---|---|---|---|
| 931 | $OCH_3$ | $OCH_3$ | O | CH | $i\text{-}C_3H_7$ | $OCH_2COON=C(CH_3)_2$ | |
| 932 | Cl | " | " | " | " | " | |
| 933 | $CH_3$ | " | " | " | " | " | |
| 934 | $NHCH_3$ | " | " | " | " | " | |
| 935 | $OCH_3$ | " | S | " | " | " | |
| 936 | Cl | " | " | " | " | " | |
| 937 | $CH_3$ | " | " | " | " | " | |
| 938 | $NHCH_3$ | " | " | " | " | " | |
| 939 | $OCH_3$ | " | " | N | " | " | |
| 940 | " | " | O | " | " | " | |
| 941 | " | " | " | " | t-Bu | " | |
| 942 | Cl | " | " | " | " | " | |
| 943 | $CH_3$ | " | " | " | " | " | |
| 944 | $NHCH_3$ | " | " | " | " | " | |
| 945 | $OCH_3$ | " | " | " | " | " | |
| 946 | Cl | " | " | " | " | " | |
| 947 | $CH_3$ | " | " | " | " | " | |
| 948 | $NHCH_3$ | " | " | " | " | " | |
| 949 | $OCH_3$ | " | " | N | " | " | |
| 950 | " | " | O | " | " | " | |
| 951 | " | " | " | CH | Cyclopentyl | " | |
| 952 | Cl | " | " | " | " | " | |
| 953 | $CH_3$ | " | " | " | " | " | |
| 954 | $NHCH_3$ | " | " | " | " | " | |
| 955 | $OCH_3$ | " | S | " | " | " | |
| 956 | Cl | " | " | " | " | " | |
| 957 | $CH_3$ | " | " | " | " | " | |
| 958 | $NHCH_3$ | " | " | " | " | " | |
| 959 | $OCH_3$ | " | " | N | " | " | |
| 960 | " | " | O | " | " | " | |

| Bsp-Nr. | $R^1$ | $R^2$ | X | Y | $R^3$ | $R^4$ | Schmp.(C°) bzw.$[n]_D^{20}$ |
|---|---|---|---|---|---|---|---|
| 961 | $OCH_3$ | $OCH_3$ | O | CH | i-$C_3H_7$ | $OCH(CH_3)CON=C(CH_3)_2$ | |
| 962 | Cl | " | " | " | " | " | |
| 963 | $CH_3$ | " | " | " | " | " | |
| 964 | $NHCH_3$ | " | " | " | " | " | |
| 965 | $OCH_3$ | " | S | " | " | " | |
| 966 | Cl | " | " | " | " | " | |
| 967 | $CH_3$ | " | " | " | " | " | |
| 968 | $NHCH_3$ | " | " | " | " | " | |
| 969 | $OCH_3$ | " | " | N | " | " | |
| 970 | " | " | O | " | " | " | |
| 971 | " | " | " | " | t-Bu | " | |
| 972 | Cl | " | " | " | " | " | |
| 973 | $CH_3$ | " | " | " | " | " | |
| 974 | $NHCH_3$ | " | " | " | " | " | |
| 975 | $OCH_3$ | " | " | " | " | " | |
| 976 | Cl | " | " | " | " | " | |
| 977 | $CH_3$ | " | " | " | " | " | |
| 978 | $NHCH_3$ | " | " | " | " | " | |
| 979 | $OCH_3$ | " | " | N | " | " | |
| 980 | " | " | O | " | " | " | |
| 981 | " | " | " | CH | Cyclopentyl | " | |
| 982 | Cl | " | " | " | " | " | |
| 983 | $CH_3$ | " | " | " | " | " | |
| 984 | $NHCH_3$ | " | " | " | " | " | |
| 985 | $OCH_3$ | " | S | " | " | " | |
| 986 | Cl | " | " | " | " | " | |
| 987 | $CH_3$ | " | " | " | " | " | |
| 988 | $NHCH_3$ | " | " | " | " | " | |
| 989 | $OCH_3$ | " | " | N | " | " | |
| 990 | " | " | O | " | " | " | |

| Bsp-Nr. | $R^1$ | $R^2$ | X | Y | $R^3$ | $R^4$ | Schmp.(C°) bzw.$[n]_D^{20}$ |
|---|---|---|---|---|---|---|---|
| 991 | $OCH_3$ | $OCH_3$ | O | CH | i-$C_3H_7$ | $-NH-CH_2-CO-OCH_2CH=CH_2$ | |
| 992 | Cl | " | " | " | " | " | |
| 993 | $CH_3$ | " | " | " | " | " | |
| 994 | $NHCH_3$ | " | " | " | " | " | |
| 995 | $OCH_3$ | " | S | " | " | " | |
| 996 | Cl | " | " | " | " | " | |
| 997 | $CH_3$ | " | " | " | " | " | |
| 998 | $NHCH_3$ | " | " | " | " | " | |
| 999 | $OCH_3$ | " | " | N | " | " | |
| 1000 | " | " | O | " | " | " | |
| 1001 | " | " | " | " | t-Bu | " | |
| 1002 | Cl | " | " | " | " | " | |
| 1003 | $CH_3$ | " | " | " | " | " | |
| 1004 | $NHCH_3$ | " | " | " | " | " | |
| 1005 | $OCH_3$ | " | " | " | " | " | |
| 1006 | Cl | " | " | " | " | " | |
| 1007 | $CH_3$ | " | " | " | " | " | |
| 1008 | $NHCH_3$ | " | " | " | " | " | |
| 1009 | $OCH_3$ | " | " | N | " | " | |
| 1010 | " | " | O | " | " | " | |
| 1011 | " | " | " | CH | Cyclopentyl | " | |
| 1012 | Cl | " | " | " | " | " | |
| 1013 | $CH_3$ | " | " | " | " | " | |
| 1014 | $NHCH_3$ | " | " | " | " | " | |
| 1015 | $OCH_3$ | " | S | " | " | " | |
| 1016 | Cl | " | " | " | " | " | |
| 1017 | $CH_3$ | " | " | " | " | " | |
| 1018 | $NHCH_3$ | " | " | " | " | " | |
| 1019 | $OCH_3$ | " | " | N | " | " | |
| 1020 | " | " | O | " | " | " | |

| Bsp-Nr. | $R^1$ | $R^2$ | X | Y | $R^3$ | $R^4$ | Schmp.(C°) bzw.$[n]_D^{20}$ |
|---|---|---|---|---|---|---|---|
| 1021 | $OCH_3$ | $OCH_3$ | O | CH | i-$C_3H_7$ | $-NH-CH_2CO-OCH_2C\equiv CH$ | |
| 1022 | Cl | " | " | " | " | " | |
| 1023 | $CH_3$ | " | " | " | " | " | |
| 1024 | $NHCH_3$ | " | " | " | " | " | |
| 1025 | $OCH_3$ | " | S | " | " | " | |
| 1026 | Cl | " | " | " | " | " | |
| 1027 | $CH_3$ | " | " | " | " | " | |
| 1028 | $NHCH_3$ | " | " | " | " | " | |
| 1029 | $OCH_3$ | " | " | N | " | " | |
| 1030 | " | " | O | " | " | " | |
| 1031 | " | " | " | " | t-Bu | " | |
| 1032 | Cl | " | " | " | " | " | |
| 1033 | $CH_3$ | " | " | " | " | " | |
| 1034 | $NHCH_3$ | " | " | " | " | " | |
| 1035 | $OCH_3$ | " | " | " | " | " | |
| 1036 | Cl | " | " | " | " | " | |
| 1037 | $CH_3$ | " | " | " | " | " | |
| 1038 | $NHCH_3$ | " | " | " | " | " | |
| 1039 | $OCH_3$ | " | " | N | " | " | |
| 1040 | " | " | O | " | " | " | |
| 1041 | " | " | " | CH | Cyclopentyl | " | |
| 1042 | Cl | " | " | " | " | " | |
| 1043 | $CH_3$ | " | " | " | " | " | |
| 1044 | $NHCH_3$ | " | " | " | " | " | |
| 1045 | $OCH_3$ | " | S | " | " | " | |
| 1046 | Cl | " | " | " | " | " | |
| 1047 | $CH_3$ | " | " | " | " | " | |
| 1048 | $NHCH_3$ | " | " | " | " | " | |
| 1049 | $OCH_3$ | " | " | N | " | " | |
| 1050 | " | " | O | " | " | " | |

| Bsp-Nr. | $R^1$ | $R^2$ | X | Y | $R^3$ | $R^4$ | Schmp.(C°) bzw.$[n]_D^{20}$ |
|---|---|---|---|---|---|---|---|
| 1051 | $OCH_3$ | $OCH_3$ | O | CH | i-$C_3H_7$ | -NHCH($CH_3$)COSCH$_2$Ph | |
| 1052 | Cl | " | " | " | " | " | |
| 1053 | $CH_3$ | " | " | " | " | " | |
| 1054 | $NHCH_3$ | " | " | " | " | " | |
| 1055 | $OCH_3$ | " | S | " | " | " | |
| 1056 | Cl | " | " | " | " | " | |
| 1057 | $CH_3$ | " | " | " | " | " | |
| 1058 | $NHCH_3$ | " | " | " | " | " | |
| 1059 | $OCH_3$ | " | " | N | " | " | |
| 1060 | " | " | O | " | " | " | |
| 1061 | " | " | " | " | t-Bu | " | |
| 1062 | Cl | " | " | " | " | " | |
| 1063 | $CH_3$ | " | " | " | " | " | |
| 1064 | $NHCH_3$ | " | " | " | " | " | |
| 1065 | $OCH_3$ | " | " | " | " | " | |
| 1066 | Cl | " | " | " | " | " | |
| 1067 | $CH_3$ | " | " | " | " | " | |
| 1068 | $NHCH_3$ | " | " | " | " | " | |
| 1069 | $OCH_3$ | " | " | N | " | " | |
| 1070 | " | " | O | " | " | " | |
| 1071 | " | " | " | CH | Cyclopentyl | " | |
| 1072 | Cl | " | " | " | " | " | |
| 1073 | $CH_3$ | " | " | " | " | " | |
| 1074 | $NHCH_3$ | " | " | " | " | " | |
| 1075 | $OCH_3$ | " | S | " | " | " | |
| 1076 | Cl | " | " | " | " | " | |
| 1077 | $CH_3$ | " | " | " | " | " | |
| 1078 | $NHCH_3$ | " | " | " | " | " | |
| 1079 | $OCH_3$ | " | " | N | " | " | |
| 1080 | " | " | O | " | " | " | |

| Bsp-Nr. | $R^1$ | $R^2$ | X | Y | $R^3$ | $R^4$ | Schmp.(C°) bzw.$[n]_D^{20}$ |
|---|---|---|---|---|---|---|---|
| 1081 | $OCH_3$ | $OCH_3$ | O | CH | i-$C_3H_7$ | -OCH(t-Bu)(CN) | 90-92 |
| 1082 | Cl | " | " | " | " | " | |
| 1083 | $CH_3$ | " | " | " | " | " | |
| 1084 | $NHCH_3$ | " | " | " | " | " | |
| 1085 | $OCH_3$ | " | S | " | " | " | |
| 1086 | Cl | " | " | " | " | " | |
| 1087 | $CH_3$ | " | " | " | " | " | |
| 1088 | $NHCH_3$ | " | " | " | " | " | |
| 1089 | $OCH_3$ | " | " | N | " | " | |
| 1090 | " | " | O | " | " | " | |
| 1091 | " | " | " | " | t-Bu | " | |
| 1092 | Cl | " | " | " | " | " | |
| 1093 | $CH_3$ | " | " | " | " | " | |
| 1094 | $NHCH_3$ | " | " | " | " | " | |
| 1095 | $OCH_3$ | " | " | " | " | " | |
| 1096 | Cl | " | " | " | " | " | |
| 1097 | $CH_3$ | " | " | " | " | " | |
| 1098 | $NHCH_3$ | " | " | " | " | " | |
| 1099 | $OCH_3$ | " | " | N | " | " | |
| 1100 | " | " | O | " | " | " | |
| 1101 | " | " | " | CH | Cyclopentyl | " | |
| 1102 | Cl | " | " | " | " | " | |
| 1103 | $CH_3$ | " | " | " | " | " | |
| 1104 | $NHCH_3$ | " | " | " | " | " | |
| 1105 | $OCH_3$ | " | S | " | " | " | |
| 1106 | Cl | " | " | " | " | " | |
| 1107 | $CH_3$ | " | " | " | " | " | |
| 1108 | $NHCH_3$ | " | " | " | " | " | |
| 1109 | $OCH_3$ | " | " | N | " | " | |
| 1110 | " | " | O | " | " | " | |

| Bsp-Nr. | $R^1$ | $R^2$ | X | Y | $R^3$ | $R^4$ | Schmp.(C°) bzw. $[n]_D^{20}$ |
|---|---|---|---|---|---|---|---|
| 1111 | $OCH_3$ | $OCH_3$ | O | CH | $i-C_3H_7$ | $-OCH(CH_3)CN$ | |
| 1112 | Cl | " | " | " | " | " | |
| 1113 | $CH_3$ | " | " | " | " | " | |
| 1114 | $NHCH_3$ | " | " | " | " | " | |
| 1115 | $OCH_3$ | " | S | " | " | " | |
| 1116 | Cl | " | " | " | " | " | |
| 1117 | $CH_3$ | " | " | " | " | " | |
| 1118 | $NHCH_3$ | " | " | " | " | " | |
| 1119 | $OCH_3$ | " | " | N | " | " | |
| 1120 | " | " | O | " | " | " | |
| 1121 | " | " | " | " | t-Bu | " | |
| 1122 | Cl | " | " | " | " | " | |
| 1123 | $CH_3$ | " | " | " | " | " | |
| 1124 | $NHCH_3$ | " | " | " | " | " | |
| 1125 | $OCH_3$ | " | " | " | " | " | |
| 1126 | Cl | " | " | " | " | " | |
| 1127 | $CH_3$ | " | " | " | " | " | |
| 1128 | $NHCH_3$ | " | " | " | " | " | |
| 1129 | $OCH_3$ | " | " | N | " | " | |
| 1130 | " | " | O | " | " | " | |
| 1131 | " | " | " | CH | Cyclopentyl | " | |
| 1132 | Cl | " | " | " | " | " | |
| 1133 | $CH_3$ | " | " | " | " | " | |
| 1134 | $NHCH_3$ | " | " | " | " | " | |
| 1135 | $OCH_3$ | " | S | " | " | " | |
| 1136 | Cl | " | " | " | " | " | |
| 1137 | $CH_3$ | " | " | " | " | " | |
| 1138 | $NHCH_3$ | " | " | " | " | " | |
| 1139 | $OCH_3$ | " | " | N | " | " | |

| Bsp-Nr. | $R^1$ | $R^2$ | X | Y | $R^3$ | $R^4$ | Schmp.(C°) bzw.$[n]_D^{20}$ |
|---|---|---|---|---|---|---|---|
| 1140 | $OCH_3$ | $OCH_3$ | O | N | Cyclopentyl | $-OCH(CH_3)CN$ | |
| 1141 | " | " | " | CH | i-Pr | 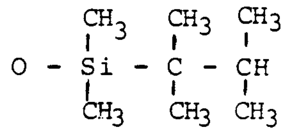 | Öl |

Biologische Beispiele

Die Schädigung der Unkrautpflanzen bzw. die Kulturpflanzenverträglichkeit wurde gemäß einem Schlüssel bonitiert, in dem die Wirksamkeit durch Wertzahlen von 0 bis 5 ausgedrückt ist. Dabei bedeutet

0 = ohne Wirkung
1 = 0 bis 20 % Wirkung bzw. Schaden
2 = 20 bis 40 % Wirkung bzw. Schaden
3 = 40 bis 60 % Wirkung bzw. Schaden
4 = 60 bis 80 % Wirkung bzw. Schaden
5 = 80 bis 100 % Wirkung bzw. Schaden

1. Unkrautwirkung im Vorauflauf

Samen bzw. Rhizomstücke von mono− und dikotylen Unkrautpflanzen wurden in Plastiktöpfen in sandiger Lehmerde ausgelegt und mit Erde abgedeckt. Die in Form von benetzbaren Pulvern oder Emulsionskonzentraten formulierten erfindungsgemäßen Verbindungen wurden dann als wäßrige Suspen− sion bzw. Emulsion mit einer Wasseraufwandmenge von umgerechnet 600 bis 800 l/ha in unterschiedlichen Dosierungen auf die Oberfläche der Abdeckerde appliziert.

Nach der Behandlung wurden die Töpfe im Gewächshaus aufgestellt und unter guten Wachstumsbedingungen für die Unkräuter gehalten. Die optische Bonitur der Pflanzen− bzw. der Auflaufschäden erfolgte nach dem Auflaufen der Versuchspflanzen nach einer Versuchszeit von 3 bis 4 Wochen im Vergleich zu unbehandelten Kontrollen. Wie die Boniturwerte in Tabelle 3 zeigen, weisen die erfindungsgemäßen Verbindungen eine gute heribzide Vorauflaufwirksamkeit gegen ein breites Spektrum von Ungräsern und Unkräutern auf.

Tabelle II

| Vorauflaufwirkung | | | | | |
|---|---|---|---|---|---|
| Bsp. – Nr. | Dosis (kg a.i./ha) | herbizide Wirkung | | | |
| | | STAL | STME | ECCR | LOMU |
| 31 | 1,25 | 5 | 5 | 5 | 5 |
| 181 | 0,3 | 5 | 5 | 5 | 5 |
| 211 | 0,3 | 5 | 5 | 5 | 5 |
| 1 | 0,3 | 5 | 5 | 5 | 5 |
| 571 | 0,3 | 5 | 5 | 5 | 5 |
| 361 | 0,3 | 5 | 5 | 5 | 5 |
| 391 | 0,3 | 5 | 5 | 4 | 4 |
| 511 | 0,3 | 5 | 5 | 5 | 5 |

Abkürzungen:
STAL = Sinapis alba
STME = Stellaria media
ECCR = Echinochloa crus – galli
LOMU = Lolium multiflorum
a.i. = Aktivsubstanz

2. Unkrautwirkung im Nachauflauf

Samen bzw. Rhizomstücke von mono – und dikotylen Unkräutern wurden in Plastiktöpfen in sandigem Lehmboden ausgelegt, mit Erde abgedeckt und im Gewächshaus unter guten Wachstumsbedingungen angezogen. Drei Wochen nach der Aussaat wurden die Versuchspflanzen im Dreiblattstadium behandelt.

Die als Spritzpulver bzw. als Emulsionskonzentrate formulierten erfindungsgemäßen Verbindungen wurden in verschiedenen Dosierungen mit einer Wasseraufwandmenge von umgerechnet 600 bis 800 l/ha auf die grünen Pflanzenteile gesprüht und nach ca. 3 bis 4 Wochen Standzeit der Versuchspflanzen im Gewächshaus unter optimalen Wachstumsbedingungen die Wirkung der Präparate optisch im Vergleich zu unbehandelten Kontrollen bonitiert.

Die erfindungsgemäßen Mittel weisen auch im Nachauflauf eine gute herbizide Wirksamkeit gegen ein breites Spektrum wirtschaftlich wichtiger Ungräser und Unkräuter auf.

Tabelle III

| Nachauflaufwirkung | | | | | |
|---|---|---|---|---|---|
| Bsp. – Nr. | Dosis (kg a.i./ha) | herbizide Wirkung | | | |
| | | STAL | STME | ECCR | LOMU |
| 31 | 1,25 | 5 | 5 | 5 | 5 |
| 181 | 0,3 | 5 | 5 | 5 | 5 |
| 211 | 0,3 | 5 | 4 | 5 | 5 |
| 1 | 0,3 | 5 | 5 | 5 | 5 |
| 571 | 0,3 | 4 | 4 | 3 | 5 |
| 361 | 0,3 | 5 | 5 | 5 | 5 |
| 391 | 0,3 | 5 | 5 | 4 | 4 |
| 511 | 0,3 | 5 | 5 | 5 | 5 |
| Abkürzungen: wie in Tabelle II | | | | | |

## Patentansprüche

**1.** Verbindungen der Formel (I)

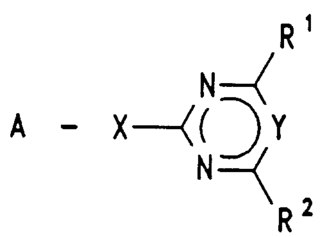

(I)

worin

A einen Rest der Formel

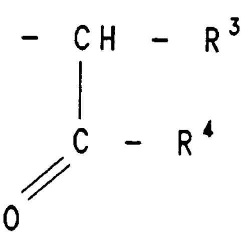

oder

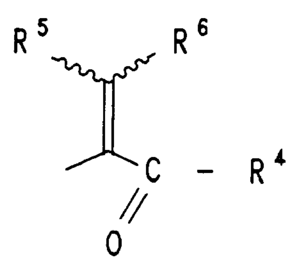

X O oder S,

Y N oder CH,

$R^1$,$R^2$ unabhängig voneinander Wasserstoff, Alkyl, Alkoxy, Alkylthio, Halogen, Haloalkoxy, Amino, Alkylamino oder Dialkylamino,

$R^3$ einen aliphatischen, araliphatischen oder aromatischen Rest der Formel

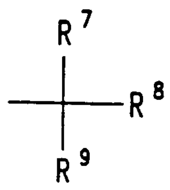 , 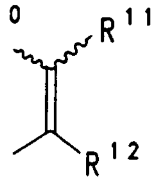 , 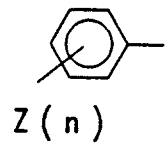 , 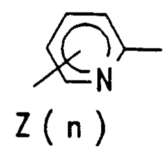

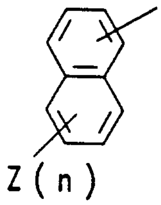 , 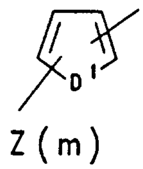 , 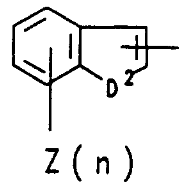 oder 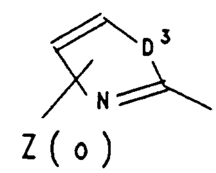

$R^5$ Alkyl, Alkenyl oder Alkinyl, wobei die letztgenannten drei Reste unabhängig voneinander unsubstituiert oder ein- oder mehrfach durch Reste aus der Gruppe Alkoxy, Alkylthio, Amino, Alkylamino, Dialkylamino, Nitro, Halogen, Phenyl und substituiertes Phenyl substituiert sind, oder
Cycloalkyl oder Cycloalkenyl, wobei die letztgenannten beiden Reste unabhängig voneinander unsubstituiert oder ein- oder mehrfach durch Reste aus der Gruppe Alkyl, Alkoxy, Alkylthio, Amino, Alkylamino, Dialkylamino, Nitro, Halogen, Phenyl und substituiertes Phenyl substituiert sind, oder
einen Rest der Formel

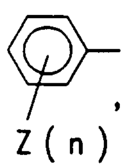 , 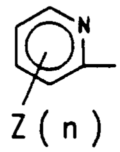 , 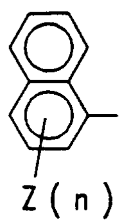 , 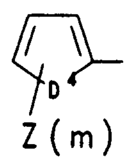 , 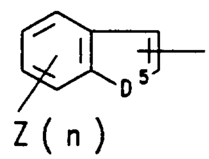

oder 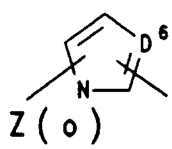 ,

$R^6$ Wasserstoff oder Alkyl,

$R^7$ Wasserstoff, Halogen, Alkyl, das unsubstituiert oder durch einen oder mehrere Reste aus der Gruppe Halogen, Hydroxy, Alkoxy und Alkylthio substituiert ist, oder Cycloalkyl, Hydroxy, Cyano, Thienyl, Naphthyl, Dihydronaphthyl, wobei die letztgenannten drei Reste unsubstituiert oder substituiert sind, oder einen Rest der Formel

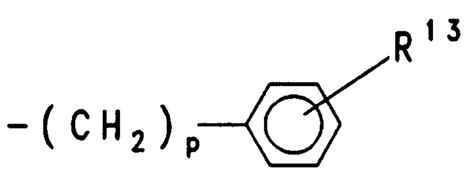

$R^{13}$ Wasserstoff, Halogen, Nitro, Alkyl, Haloalkyl, Alkoxy, Alkylsulfonyl, Alkylsulfinyl oder

Alkylthio,

$R^8$,$R^9$ unabhängig voneinander Wasserstoff oder Alkyl oder $R^8$ und $R^9$ gemeinsam mit dem verbundenen C – Atom einen 3 – bis 6 – gliedrigen Ring, der ein Sauerstoff – atom enthalten kann und durch ein oder mehrere Alkylgruppen substituiert sein kann,

$R^{10}$ unabhängig voneinander Wasserstoff oder Alkyl oder $R^{10}$ gemeinsam mit $R^{12}$ und der verbundenen Ethenylen – Gruppe einen Cyclopenten – oder Cyclohexenring, der unsubstituiert oder durch ein oder mehrere Alkylgruppen substituiert ist,

$R^{11}$ Wasserstoff oder Alkyl,

$R^{12}$ Alkyl, Phenyl oder substituiertes Phenyl oder $R^{12}$ gemeinsam mit $R^{10}$ und der verbundenen Ethenylen – Gruppe einen Cyclopenten – oder Cyclohexenring, der unsubstituiert oder durch ein oder mehrere Alkylgruppen substituiert ist,

$D^1$ bis $D^6$ unabhängig voneinander O, S oder $NR^{14}$,

$R^{14}$ Wasserstoff, Alkyl oder Phenyl,

n eine ganze Zahl von 0 bis 4,

m eine ganze Zahl von 0 bis 3,

o eine ganze Zahl von 0 bis 2,

p eine ganze Zahl von 0 bis 2,

Z unabhängig voneinander Halogen, Alkyl, Haloalkyl, Alkoxy, Haloalkoxy, Alkylthio, Amino, Alkylamino, Dialkylamino, Nitro, Phenyl oder substituiertes Phenyl,

$R^4$ einen Alkoxyrest, der durch Alkenyloxy, Alkinyloxy, Cycloalkyloxy, Cycloalkenyloxy, Benzyloxy, Benzylthio, $(R^*)_3Si$ oder $(R^*)_3SiO$, worin die Reste $R^*$ unabhängig voneinander für Alkyl, Alkenyl oder Alkinyl, Aryl oder substituiertes Aryl stehen, oder Cyano, Nitro, Alkylcarbonyl, Alkenylcarbonyl, Alkinylcarbonyl, Cycloalkylcar – bonyl, Alkylsulfonyl, Alkylsulfinyl, Dialkylphosphinyl, Dialkylphosphonyl, Dialkyl – phosphoryl, Pyridyl oder substituiertes Pyridyl substituiert ist, oder
$R^4$ den Rest eines 5 – oder 6 – Ringlactons oder
$R^4$ einen Rest der Formel

$- D^7 - CR^{15}R^{16} - D^8 - L$
$- NR^{32} - SO_2 - R^{33}$
$- NR^{34} - O - R^{35}$
$- NH - N = CR^{21}R^{22}$ oder

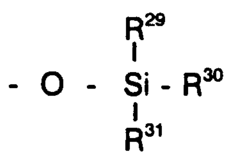

$D^7$ O, S oder $NR^{14}$,

$D^8$ eine direkte Bindung oder eine gesättigte oder ungesättigte Kohlenwasserstoff – gruppe,

$R^{15}$, $R^{16}$ unabhängig voneinander Wasserstoff, Alkyl, Alkenyl, Alkinyl, Aryl, substituiertes Aryl, Benzyl, Alkoxy, Alkenyloxy, Alkinyloxy, Aryloxy oder substituiertes Aryloxy,

L einen Säurederivat – Rest der Formel

$- CO - O - R^{17}$
$- CO - S - R^{18}$
$- CO - NR^{19}R^{20}$
$- CO - O - N = CR^{21}R^{22}$
$- CO - N = CR^{21}R^{22}$
$- O - CO - R^{23}$
$- S - CO - R^{23}$
$- NR^{14} - CO - R^{23}$

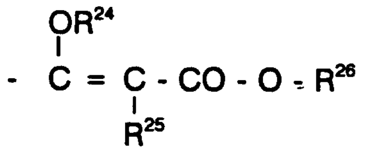 oder

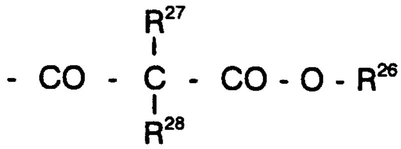

$R^{17}$, $R^{18}$ Wasserstoff, Alkyl, Alkenyl oder Alkinyl, wobei die letztgenannten 3 Reste unsubstituiert oder durch einen oder mehrere Reste aus der Gruppe Halogen substituiert sind, Cycloalkyl, Cycloalkenyl, Benzyl, Phenyl oder substituiertes Phenyl,

$R^{19}$, $R^{20}$ unabhängig voneinander Wasserstoff, Alkyl, Alkenyl, Alkinyl, Cycloalkyl, Benzyl, Phenyl oder substituiertes Phenyl oder gemeinsam mit dem verbundenen N-Atom einen heterocyclischen 3-bis 7-gliedrigen Ring, der neben dem N-Atom noch Heteroatome aus der Gruppe N, O und S enthalten kann,

$R^{21}$, $R^{22}$ unabhängig voneinander Wasserstoff, Alkyl, Alkenyl oder Alkinyl oder gemeinsam mit dem verbundenen C-Atom einen carbocylischen 4-bis 8-gliedrigen Rest,

$R^{23}$ Wasserstoff, Alkyl, Cycloalkyl, Phenyl oder substituiertes Phenyl,

$R^{24}$ Alkyl, Alkenyl oder Alkinyl, wobei die letztgenannten 3 Reste unsubstituiert oder durch einen oder mehrere Reste aus der Gruppe Halogen, Alkoxy, Alkenyloxy und Alkinyloxy substituiert sind, oder Cycloalkyl, Cycloalkenyl, Alkanoyl, Alkoxycarbonyl, Benzyl, Benzoxycarbonyl, Phenyl oder Benzoyl, wobei die letztgenannten 4 Reste unsubstituiert oder substituiert sind,

$R^{25}$ Wasserstoff, Alkyl, Alkanoyl, Alkoxycarbonyl, Alkenyloxycarbonyl, Alkinyloxycarbonyl, Phenyl oder substituiertes Phenyl,

$R^{26}$ Wasserstoff, Alkyl, Alkenyl oder Alkinyl, wobei die letztgenannten 3 Reste unsubstituiert oder durch ein oder mehrere Reste aus der Gruppe Halogen substituiert sind,

$R^{27}$, $R^{28}$ unabhängig voneinander Wasserstoff, Alkyl, Alkanoyl, Alkoxycarbonyl, Alkenyloxycarbonyl, Alkinyloxycarbonyl, Phenyl oder substituiertes Phenyl,

$R^{29}$, $R^{30}$, $R^{31}$ unabhängig voneinander Alkyl, Alkenyl oder Alkinyl, Phenyl oder substituiertes Phenyl,

$R^{32}$ Wasserstoff, Alkyl, Alkenyl, Alkinyl, Aryl oder Aralkyl,

$R^{33}$ Alkyl, Aryl, substituiertes Aryl, Aralkyl oder substituiertes Aralkyl,

$R^{34}$ Wasserstoff, Alkyl, Alkenyl, Alkinyl, Aryl oder Aralkyl und

$R^{35}$ Wasserstoff, Alkyl, Alkenyl, Alkinyl, Aryl oder Aralkyl

bedeuten.

**2.** Verbindungen nach Anspruch 1, dadurch gekennzeichnet, daß $R^1$ und $R^2$ unabhängig voneinander $C_1-C_4$-Alkyl, $C_1-C_4$-Alkoxy, Halogen, $C_1-C_4$-Haloalkoxy, $C_1-C_4$-Alkylamino oder Di-($C_1-C_4$-alkyl)amino bedeuten.

**3.** Verbindungen nach Anspruch 1 oder 2, dadurch gekennzeichnet, daß

A einen Rest der Formel $-CHR^3-CO-R^4$

$R^3$ einen aliphatischen, araliphatischen oder aromatischen Rest der Formel

$-CR^7R^8R^9$ oder

Phenyl, Pyridyl oder Thienyl, wobei die letztgenannten 3 Reste unsubstituiert oder durch 1 bis 3 Reste Z substituiert sind,

$R^7$ Wasserstoff, Halogen, $C_1-C_4$-Alkyl, Phenyl oder Benzyl,

$R^8$, $R^9$ unabhängig voneinander Wasserstoff oder $C_1-C_4$-Alkyl oder $R^8$ und $R^9$ gemeinsam mit

dem verbundenen C – Atom einen 5 – oder 6 – gliedrigen Ring und

Z unabhängig voneinander Halogen, $C_1 - C_4$ – Alkyl, $C_1 - C_4$ – Haloalkyl, $C_1 - C_4$ – Alkoxy, $C_1 - C_4$ – Haloalkoxy, $C_1 - C_4$ – Alkylthio, $C_1 - C_4$ – Alkylamino, Di – ($C_1 - C_4$ – alkyl) – amino oder Nitro

bedeuten.

**4.** Verbindungen nach Anspruch 1 oder 2, dadurch gekennzeichnet, daß

A einen Rest der Formel

$$R^5R^6C = \overset{|}{C} - CO - R^4$$

$R^5$ $C_1 - C_4$ – Alkyl, $C_2 - C_4$ – Alkenyl oder $C_2 - C_4$ – Alkinyl, Cyclohexyl, Cyclopentyl, Phenyl, Pyridyl oder Thienyl, wobei die letztgenannten 3 Reste unsubstituiert oder durch 1 bis 3 Reste Z substituiert sind,

$R^6$ Wasserstoff oder $C_1 - C_4$ – Alkyl und

Z unabhängig voneinander Halogen, $C_1 - C_4$ – Alkyl, $C_1 - C_4$ – Haloalkyl, $C_1 - C_4$ – Alkoxy, $C_1 - C_4$ – Haloalkoxy, $C_1 - C_4$ – Alkylthio, $C_1 - C_4$ – Alkylamino, Di – ($C_1 - C_4$ – alkyl) – amino oder Nitro

bedeuten.

**5.** Verbindungen nach einem der Ansprüche 1 bis 4, dadurch gekennzeichnet, daß

$R^4$ einen $C_1 - C_4$ – Alkoxyrest, der durch $C_2 - C_4$ – Alkenyloxy, $C_2 - C_4$ – Alkinyloxy, Cyclohexyloxy, Cyclohexenyloxy, Cyclopentyloxy, Cyclopentenyloxy, Benzyloxy, Benzylthio, $(R^*)_3Si$ oder $(R^*)_3SiO$, worin die Reste $R^*$ unabhängig voneinander für $C_1 - C_4$ – Alkyl, $C_2 - C_4$ – Alkenyl, $C_2 - C_4$ – Alkinyl oder Phenyl stehen, Cyano, Nitro, ($C_1 - C_4$ – Alkyl) – carbonyl, $C_1 - C_4$ – Alkylsulfonyl, $C_1 - C_4$ – Alkylsulfinyl, Di – ($C_1 - C_4$ – alkyl) – phosphinyl, Di – ($C_1 - C_4$ – alkyl) – phosphonyl, Di – ($C_1 - C_4$ – alkyl)phosphoryl, Pyridyl substituiert ist, oder

$R^4$ den Rest eines 5 – oder 6 – Ringlactons, der die Bindung zum übrigen Molekülteil in $\alpha$ – Position zur Carbonylgruppe hat, oder

$R^4$ einen Rest der Formel

$$- D^7 - CR^{15}R^{16} - D^8 - L$$

$D^7$ O, S, NH, Methylamino oder Ethylamino,

$D^8$ eine direkte Bindung,

$R^{15}$, $R^{16}$ unabhängig voneinander Wasserstoff, $C_1 - C_4$ – Alkyl, $C_2 - C_4$ – Alkenyl, $C_2 - C_4$ – Alkinyl, Phenyl oder Benzyl,

L einen Säurederivat – Rest der Formel

$- CO - O - R^{17}$

$- CO - S - R^{18}$

$- CO - NR^{19}R^{20}$

$- CO - O - N = CR^{21}R^{22}$

$- CO - N = CR^{21}R^{22}$

$- O - CO - R^{23}$

$- S - CO - R^{23}$

$- NR^{14} - CO - R^{23}$

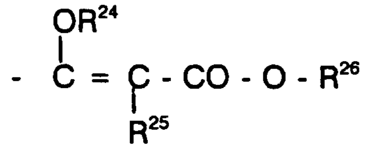 oder

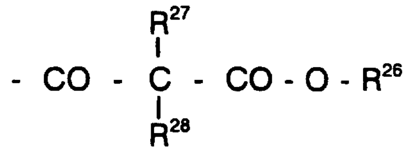

$R^{17}$ Wasserstoff, $C_2-C_4$-Alkenyl oder $C_2-C_4$-Alkinyl, Cyclohexyl, Cyclopentyl, Cyclohexenyl, Cyclopentenyl, Benzyl, Phenyl oder substituiertes Phenyl,

$R^{18}$ Wasserstoff, $C_1-C_4$-Alkyl, $C_2-C_4$-Alkenyl oder $C_2-C_4$-Alkinyl, Cyclohexyl, Cyclopentyl, Cyclohexenyl, Cyclopentenyl, Benzyl, Phenyl oder substituiertes Phenyl,

$R^{19}$, $R^{20}$ unabhängig voneinander Wasserstoff, $C_1-C_4$-Alkyl, $C_2-C_4$-Alkenyl, $C_2-C_4$-Alkinyl, Cyclohexyl, Cyclopentyl, Benzyl, Phenyl oder substituiertes Phenyl oder gemeinsam mit dem verbundenen N-Atom einen heterocyclischen 5- oder 6-gliedrigen Ring, der neben dem N-Atom noch ein O-Atom als Heteroatom enthalten kann,

$R^{21}$, $R^{22}$ unabhängig voneinander $C_1-C_4$-Alkyl, $C_2-C_4$-Alkenyl oder $C_2-C_4$-Alkinyl oder gemeinsam mit dem verbundenen C-Atom einen carbocylischen 5- bis 6-gliedrigen Rest,

$R^{23}$ Wasserstoff, $C_1-C_4$-Alkyl, Cyclohexyl, Cyclopentyl, Phenyl oder substituiertes Phenyl,

$R^{24}$ $C_1-C_4$-Alkyl, $C_2-C_4$-Alkenyl oder $C_2-C_4$-Alkinyl, wobei die letztgenannten 3 Reste unsubstituiert oder durch einen oder mehrere Reste aus der Gruppe Halogen oder $C_1-C_4$-Alkoxy substituiert sind, oder $C_1-C_4$-Alkanoyl, $(C_1-C_4$-Alkoxy)-carbonyl, Benzyl, Benzoxycarbonyl, Phenyl oder Benzoyl, wobei die letztgenannten 4 Reste unsubstituiert oder durch $C_1-C_4$-Alkyl, $C_1-C_4$-Alkoxy, Halogen oder Nitro substituiert sind,

$R^{25}$ Wasserstoff oder $C_1-C_4$-Alkyl,

$R^{26}$ Wasserstoff, $C_1-C_4$-Alkyl, $C_2-C_4$-Alkenyl oder $C_2-C_4$-Alkinyl, wobei die letztgenannten 3 Reste unsubstituiert oder durch einen oder mehrere Reste aus der Gruppe Halogen substituiert sind und

$R^{27}$, $R^{28}$ unabhängig voneinander Wasserstoff oder $C_1-C_4$-Alkyl

bedeuten.

**6.** Verbindungen nach einem der Ansprüche 1 bis 5, dadurch gekennzeichnet, daß

$R^4$ einen $C_1-C_4$-Alkoxyrest, der durch Allyl, Propargyl, $(R^*)_3Si$ oder $(R^*)_3SiO$, worin die Reste R* unabhängig voneinander für Methyl, Ethyl oder Phenyl stehen, oder Cyano, $C_1-C_4$-Alkylsulfonyl, $C_1-C_4$-Alkylsulfinyl, Di-$(C_1-C_4$-alkyl)-phosphinyl, Di-($C_1-C_4$-alkyl)-phosphonyl, Di-$(C_1-C_4$-alkyl)-phosphoryl oder Pyridyl substituiert ist, oder

$R^4$ den Rest eines 5-Ringlactons, der die Bindung zum übrigen Molekülteil in $\alpha$-Position zur Carbonylgruppe hat, oder

$R^4$ einen Rest der Formel

$$-D^7-CR^{15}R^{16}-D^8-L$$

$D^7$ O, S oder NH,

$D^8$ eine direkte Bindung,

$R^{15}$, $R^{16}$ unabhängig voneinander Wasserstoff oder Methyl,

L einen Säurederivat-Rest der Formel

$-CO-O-R^{17}$

$-CO-S-R^{18}$

$-CO-N=CR^{21}R^{22}$

$-CO-O-N=CR^{21}R^{22}$

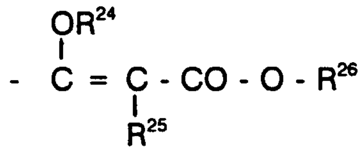 oder

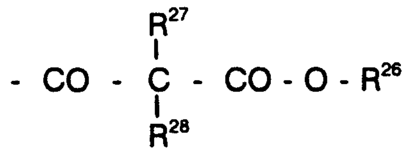

| | |
|---|---|
| $R^{17}$ | Wasserstoff, $C_2-C_4$-Alkenyl oder $C_2-C_4$-Alkinyl, Benzyl oder Phenyl, |
| $R^{18}$ | Wasserstoff, $C_1-C_4$-Alkyl, $C_2-C_4$-Alkenyl oder $C_2-C_4$-Alkinyl, Benzyl oder Phenyl, |
| $R^{19}$, $R^{20}$ | unabhängig voneinander Wasserstoff, $C_1-C_4$-Alkyl, $C_2-C_4$-Alkenyl, $C_2-C_4$-Alkinyl, Cyclohexyl, Cyclopentyl, Benzyl, Phenyl oder substituiertes Phenyl oder gemeinsam mit dem verbundenen N-Atom einen heterocyclischen 5- oder 6-gliedrigen Ring, der neben dem N-Atom noch ein O-Atom als Heteroatom enthalten kann, |
| $R^{24}$ | $C_1-C_4$-Alkyl, wobei der letztgenannte Rest unsubstituiert oder durch einen oder mehrere Reste aus der Gruppe Halogen oder $C_1-C_4$-Alkoxy substituiert ist, oder $C_1-C_4$-Alkanoyl, ($C_1-C_4$-Alkoxy)-carbonyl, Benzyl, Benzoxycarbonyl, Phenyl oder Benzoyl, wobei die letztgenannten 4 Reste unsubstituiert oder durch $C_1-C_4$-Alkyl, $C_1-C_4$-Alkoxy, Halogen oder Nitro substituiert sind, |
| $R^{25}$ | Wasserstoff oder $C_1-C_4$-Alkyl, |
| $R^{26}$ | Wasserstoff, $C_1-C_4$-Alkyl, $C_2-C_4$-Alkenyl oder $C_2-C_4$-Alkinyl und |
| $R^{27}$, $R^{28}$ | unabhängig voneinander Wasserstoff oder $C_1-C_4$-Alkyl |

bedeuten.

**7.** Verbindungen nach einem der Ansprüche 1 bis 4, dadurch gekennzeichnet, daß
$R^4$ einen Rest der Formel

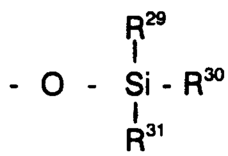

bedeutet, worin $R^{29}$, $R^{30}$, $R^{31}$ unabhängig voneinander $C_1-C_4$-Alkyl, $C_2-C_4$-Alkenyl oder $C_2-C_4$-Alkinyl, Phenyl oder substituiertes Phenyl bedeuten.

**8.** Verbindungen nach Anspruch 7, dadurch gekennzeichnet, daß $R^{29}$, $R^{30}$, $R^{31}$ unabhängig voneinander $C_1-C_4$-Alkyl, $C_2-C_4$-Alkenyl oder $C_2-C_4$-Alkinyl oder Phenyl bedeuten.

**9.** Verfahren zur Herstellung einer nach einem der Ansprüche 1 bis 8 definierten Verbindungen der Formel (I), dadurch gekennzeichnet, daß man
(a) falls A ein Rest der Formel $-CHR^3-CO-R^4$ ist, eine Verbindung der Formel (II)

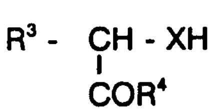 (II),

worin $R^3$, $R^4$ und X die bei Formel (I) genannten Bedeutungen haben,
mit einer Verbindung der Formel (III)

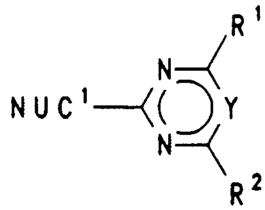

(III),

worin $NUC^1$ eine Abgangsgruppe bedeutet und $R^1$, $R^2$ und Y die bei Formel (I) genannte Bedeutung haben,
in Gegenwart einer anorganischen oder organischen Base umsetzt,
(b) falls A ein Rest der Formel $-CHR^3-CO-R^4$ ist, eine Verbindung der Formel (IV)

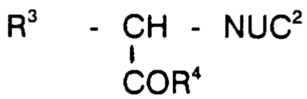

(IV),

worin $NUC^2$ eine Abgangsgruppe bedeutet und $R^3$ und $R^4$ wie bei Formel (I) definiert sind,
mit einer Verbindung der Formel (V)

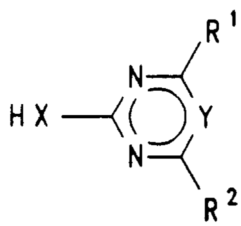

(V),

worin $R^1$, $R^2$, X und Y die bei Formel (I) genannten Bedeutungen haben, in Gegenwart einer anorganischen oder organischen Base umsetzt,
(c) falls A die Gruppe

$$R^5R^6C = C\text{-}CO\text{-}R^4$$

ist, eine Verbindung der Formel (VI)

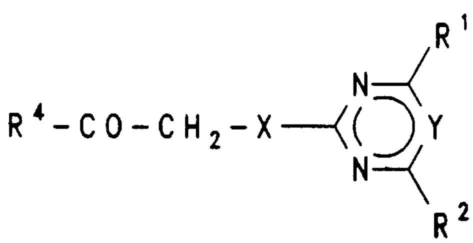

(VI),

worin $R^1$, $R^2$, $R^4$, X und Y die bei Formel (I) genannten Bedeutungen haben,
mit einem Aldehyd oder Keton der Formel (VII)

$R^5 - CO - R^6$ (VII),

worin $R^5$ und $R^6$ die bei Formel (I) genannten Bedeutungen haben, in Gegenwart einer geeigneten

anorganischen oder organischen Base umsetzt,
(d) falls A ein Rest der Formel $-CHR^3-CO-R^4$ ist, eine Verbindung der unter (c) genannten Formel (VI) mit einer Halogenverbindung der Formel (VIII)

$R^3$ − Hal (VIII),

worin Hal Chlor, Brom oder Iod bedeutet und $R^3$ wie bei Formel (I) definiert ist, in Gegenwart einer organischen oder organischen Base umsetzt oder
(e) eine Verbindung der Formel (IX) bzw. (X)

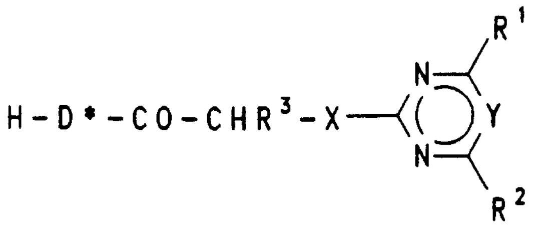

(IX)

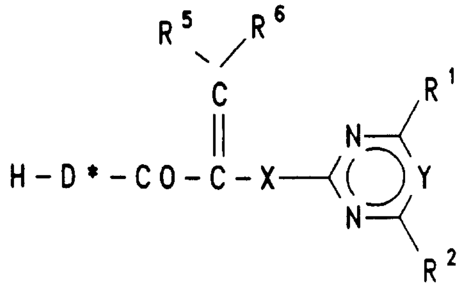

(X)

worin $R^1$, $R^2$, $R^3$, $R^5$, $R^6$, X und Y wie bei Formel (I) definiert sind und D* O, S oder $NR^{14}$ mit $R^{14}$ = H, Alkyl oder Phenyl bedeutet, mit einer Halogenverbindung der Formel (XI)

$R^o$ − Hal (XI)

worin $R^o$ so definiert ist, daß $R^o-D^*-$ die Bedeutung von $R^4$ nach Formel (I) hat, in Gegenwart eine anorganischen oder organischen Base umsetzt oder
(f) eine Verbindung der Formel (IX) oder (X), in der D* ein Sauerstoffatom ist mit Thionylchlorid, Oxalylchlorid, Chlorcarbonat, Carbonyldiimidazol oder Dicyclohexylcarbodiimid/4−N,N−Dimethylaminopyridin in an sich bekannter Weise zu einem aktivierten Carbonsäurederivat umsetzt und letzteres mit einer Verbindung der Formel (XII)

$R^4$ − H (XII)

in Gegenwart einer anorganischen oder organischen Base umsetzt.
10. Herbizide und pflanzenwachstumsregulierende Mittel, dadurch gekennzeichnet, daß sie eine Verbindung der Formel (I) nach einem der Ansprüche 1 bis 8 und übliche Formulierungshilfsmittel enthalten.
11. Verwendung der Verbindungen der Formel (I) nach einem der Ansprüche 1 bis 8 als Herbizide oder Pflanzenwachstumsregulatoren.
12. Verfahren zur Bekämpfung von Schadpflanzen oder zur Wachstumsregulierung von Pflanzen, dadurch gekennzeichnet, daß man eine oder mehrere Verbindungen der Formel (I) nach einem der Ansprüche 1 bis 8 auf die Pflanzen, Pflanzensamen oder die Anbaufläche appliziert.

Europäisches Patentamt

# EUROPÄISCHER RECHERCHENBERICHT

Nummer der Anmeldung

EP 92 11 8813

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | EP-A-0 411 706 (SHELL)<br>* das ganze Dokument *<br>--- | 1,5-12 | C07D239/60<br>C07D251/30<br>C07D401/12 |
| X | CHEMICAL ABSTRACTS, vol. 117, no. 28, 1992, Columbus, Ohio, US;<br>abstract no. 48615r,<br>Seite 943 ;Spalte 1 ;<br>* Zusammenfassung * | 1,6-12 | C07D405/12<br>C07F7/18<br>C07F7/08<br>C07F9/6506<br>C07F9/6521 |
| X | & JP-A-03 240 777 (UBE INDUSTRIES)<br>28. Oktober 1991<br>--- | 1,6-12 | A01N43/54 |
| D,A | EP-A-0 347 811 (KUMIAI CHEMICAL INDUSTRY)<br>* das ganze Dokument *<br>--- | 1,5-12 | |
| D,A | EP-A-0 400 741 (SHELL)<br>* das ganze Dokument *<br>----- | 1,5-12 | |
| | | | RECHERCHIERTE SACHGEBIETE (Int. Cl.5) |
| | | | C07D<br>C07F |

Der vorliegende Recherchenbericht wurde für alle Patentansprüche erstellt

| Recherchenort | Abschlußdatum der Recherche | Prüfer |
|---|---|---|
| DEN HAAG | 04 FEBRUAR 1993 | FRANCOIS J.C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)